(19) Europäisches Patentamt
European Patent Office
Office européen des brevets

(11) EP 1 723 943 A1

(12) **DEMANDE DE BREVET EUROPEEN**

(43) Date de publication:
**22.11.2006 Bulletin 2006/47**

(21) Numéro de dépôt: **06300476.6**

(22) Date de dépôt: **16.05.2006**

(51) Int Cl.:
***A61K 8/04*** (2006.01) ***A61Q 17/00*** (2006.01)
***A61Q 17/04*** (2006.01) ***A61K 8/891*** (2006.01)
***A61K 8/81*** (2006.01) ***A61K 8/88*** (2006.01)
***A61K 8/40*** (2006.01)

(84) Etats contractants désignés:
**AT BE BG CH CY CZ DE DK EE ES FI FR GB GR HU IE IS IT LI LT LU LV MC NL PL PT RO SE SI SK TR**
Etats d'extension désignés:
**AL BA HR MK YU**

(30) Priorité: **17.05.2005 FR 0551270**

(71) Demandeur: **L'ORÉAL**
**75008 Paris (FR)**

(72) Inventeurs:
- **Biatry, Bruno**
**94300 Vincennes (FR)**
- **Simonnet, Jean-Thierry**
**94240 Cachan (FR)**

(74) Mandataire: **Le Coupanec, Pascale A.M.P. et al**
**Nony & Associés,**
**3 rue de Penthièvre**
**75008 Paris (FR)**

(54) **Particules d'huile gélifiée comportant au moins un filtre solaire hydrophobe.**

(57) La présente invention concerne des particules huileuses calibrées comprenant au moins un filtre solaire hydrophobe et comprenant au moins une phase huileuse structurée par au moins un polymère gélifiant, caractérisée en ce qu'elles possèdent une taille moyenne inférieure ou égale à 20 μm, en ce que ladite phase huileuse structurée possède un point de fusion supérieur ou égal à 40 °C et en ce que leur indice de circularité est compris entre 0,9 et 1.

EP 1 723 943 A1

## Description

La présente invention concerne des particules huileuses calibrées et sphériques comportant un filtre solaire hydrophobe.

La présente invention concerne également une composition cosmétique ou dermatologique comprenant ces particules, ainsi qu'un procédé de fabrication de ces particules.

Ces particules sont en particulier utiles en tant qu'agent permettant d'augmenter le facteur de protection solaire (SPF).

On sait que les radiations lumineuses de longueurs d'onde comprises entre 280 nm et 400 nm permettent le brunissement de l'épiderme humain et que les rayons de longueurs d'onde comprises entre 280 nm et 320 nm, connus sous la dénomination d'UV-B provoquent des érythèmes et des brûlures cutanées qui peuvent nuire au développement du bronzage naturel.

On sait également que les rayons UV-A, de longueurs d'onde comprises entre 320 nm et 400 nm, qui provoquent le brunissement de la peau, sont susceptibles d'induire une altération de celle-ci, notamment dans le cas d'une peau sensible ou d'une peau continuellement exposée au rayonnement solaire. Les rayons UV-A provoquent en particulier une perte d'élasticité de la peau et l'apparition de rides conduisant à un vieillissement prématuré. Ils favorisent le déclenchement de la réaction érythémateuse ou amplifient cette réaction chez certains sujets et peuvent même être à l'origine de réactions phototoxiques ou photo-allergiques.

Pour filtrer les rayons UVA et UVB, il existe sur le marché différents types de filtres solaires : les pigments et les filtres chimiques (ou filtres UV organiques). Ces filtres solaires doivent pouvoir absorber ou bloquer les rayons nocifs du soleil tout en restant inoffensifs pour l'utilisateur.

L'objectif dans la mise au point de compositions comprenant un filtre solaire est généralement d'obtenir le meilleur rapport taux de filtres solaires/efficacité. Pour cela, on utilise très régulièrement des "booster de SPF" comme par exemples les cires, des polymères gélifiants de la phase grasse. Cependant, ces composés augmentent la viscosité de la totalité de la phase grasse et de fait, celle de l'émulsion finale. Il est alors impossible d'obtenir des suspensions fluides et vaporisables. De plus, ces solutions techniques conditionnent la mise en émulsion à haute température de la totalité de la composition, ce qui s'avère préjudiciable pour les actifs thermosensibles éventuellement présents dans les compositions pour la photoprotection.

L'efficacité d'une composition antisolaire pour la peau se traduit généralement en terme de facteur de protection solaire (SPF) qui est défini par le rapport de la quantité d'énergie nécessaire pour provoquer un début d'érythème sur la peau protégée par l'agent filtrant les radiations UV sur la quantité d'énergie nécessaire pour provoquer un début d'érythème sur la peau non protégée.

Des particules de nature huileuse ont été proposées à titre d'alternative.

Ainsi, l'usage de particules d'un diamètre d'environ 50 μm à 10 mm et comportant une huile structurée par un agent de gélification pour formuler une composition de nettoyage de la peau et des cheveux a été proposée (voir US 6,737,394).

De WO 02/092043, est connue une composition de soin pour la peau comportant une phase aqueuse dans laquelle est dispersée une phase huileuse structurée à l'aide d'un agent gélifiant. La phase huileuse, dont la viscosité ne dépasse pas 5000 Pa.s, y est dispersée sous la forme de particules d'une taille variant de 1 à 500 μm.

De EP 0 375 520, est connue une composition cosmétique pour application topique comportant des particules de corps gras, comprenant un produit actif, et présentant un diamètre compris entre 3 et 10 μm.

Toutefois, à nouveau, l'ensemble des particules précédemment décrites ne proposent pas de substances actives de type filtre solaire et/ou sont obtenues au moyen de procédés ne permettant pas la production de particules d'une part calibrées dans un intervalle de taille prédéterminé.

Par ailleurs, des compositions plus particulièrement destinées à la protection de la peau et/ou des cheveux contre les rayonnements UV sont décrites dans EP 1 331 000. Ces compositions comprennent au moins une phase grasse liquide, au moins un filtre UV organique et au moins une polymère semi-cristallin solide à température ambiante et ayant une température de fusion inférieure à 70 °C. Cependant, aucune particule huileuse gélifiée et calibrée, ni aucune dispersion desdites particules huileuses gélifiées et calibrées ne sont divulguées ni encore moins leur intérêt en terme d'amélioration du SPF.

Les documents US 2004/0042980 et US 2004/0247549 décrivent des émulsions contenant un polymère structurant la phase huileuse, de préférence de type polyamide alkylé, et contenant en outre un filtre solaire. De la même façon que pour le document cité précédemment, aucune mention n'est faite de particules huileuses gélifiés et calibrées.

Il existe par conséquent un besoin pour optimiser le dépôt de filtres solaires sur la peau et/ou les cheveux en vue d'améliorer le facteur de protection solaire (SPF).

Il existe encore un besoin pour obtenir des particules huileuses comprenant un filtre solaire structurées, qui soient stables et qui n'exsudent pas dans le temps.

Il existe encore un besoin pour obtenir des particules huileuses comprenant un filtre solaire qui soient homogènes dans leur structure et leur distribution de taille.

Il existe également un besoin pour obtenir des particules huileuses comprenant un filtre solaire dont l'intégrité est maintenue lors de l'application sur un support.

Il existe en outre un besoin pour obtenir des compositions comprenant un filtre solaire comprenant en outre des actifs thermosensibles, ces actifs thermosensibles n'ayant pas à subir d'étape de mise en émulsion à haute température de la composition dans sa totalité.

Il existe enfin un besoin pour obtenir des compositions comprenant un filtre solaire qui présentent la caractéristique d'être fluides.

La présente invention a précisément pour objet de satisfaire tout ou partie des besoins en s'affranchissant des inconvénients précédemment évoqués.

Les inventeurs ont observés qu'il était possible d'obtenir à partir d'une phase huileuse structurée par au moins un polymère gélifiant des particules huileuses calibrées et sphériques comprenant au moins un filtre solaire hydrophobe qui soient conformées de manière à optimiser le dépôt à la surface de la peau et à augmenter le facteur de protection solaire.

La présente invention, selon un des ses premiers aspects a ainsi pour objet des particules huileuses calibrées et sphériques comprenant au moins un filtre solaire hydrophobe et comprenant au moins une phase huileuse structurée par au moins un polymère gélifiant, lesdites particules possédant une taille moyenne inférieure ou égale à 20 $\mu$m et ladite phase huileuse structurée possédant un point de fusion supérieur ou égal à 40 °C, et leur indice de circularité étant compris entre 0,9 et 1.

La présente invention à également pour objet des particules huileuses calibrées et sphériques comprenant au moins un filtre solaire hydrophobe et au moins une phase huileuse structurée par au moins un polymère gélifiant et possédant une taille moyenne inférieure ou égale à 20 $\mu$m, le polymère gélifiant étant choisi parmi un polymère semi-cristallin, un polyamide, un polyamide siliconé, un monoalkylester ou un polyalkylester de polysaccharide, un copolymère dibloc et/ou tribloc et/ou multibloc et/ou radial-bloc, et leurs mélanges et leur indice de circularité étant compris entre 0,9 et 1.

La présente invention, selon un autre de ses aspects, a également pour objet des particules huileuses calibrées et sphériques comprenant au moins un filtre solaire hydrophobe obtenues à partir d'une phase huileuse structurée par au moins un polymère gélifiant, le polymère gélifiant étant de nature et/ou à une teneur suffisante pour conférer à ladite phase huileuse une viscosité supérieure ou égale à 750 Pa.s au cisaillement de 1 $s^{-1}$, à 25 °C et leur indice de circularité étant compris entre 0,9 et 1.

Au sens de la présente invention, on entend désigner par "teneur suffisante", la teneur minimale nécessaire pour observer l'effet attendu, à savoir dans le cas du polymère gélifiant, l'obtention d'une phase huileuse structurée présentant la viscosité requise à l'obtention de particules conformes à la présente invention.

Dans la suite de la présente description, on entend par "filtre UV hydrophobe" tout agent filtrant les radiations UV dont la solubilité dans l'eau à 25 °C n'excède pas 0,5 %.

Les particules calibrées et sphériques selon l'invention peuvent comprendre au moins un filtre solaire.

Selon encore un autre de ses aspects, la présente invention a pour objet une dispersion en phase aqueuse et/ou hydrosoluble comprenant des particules conformes à la présente invention.

Selon encore un autre de ses aspects, la présente invention a également pour objet un procédé de fabrication d'une dispersion conforme à l'invention.

Selon encore un autre de ses aspects, la présente invention a pour objet une composition cosmétique ou dermatologique comprenant au moins des particules et/ou au moins une dispersion conforme à l'invention.

Selon encore un autre de ses aspects, la présente invention a également pour objet l'utilisation de particules et/ou au moins d'une dispersion conforme à l'invention dans une composition cosmétique destinée à la photoprotection.

Selon encore un autre de ses aspects, la présente invention a également pour objet un procédé de maquillage et/ou de soin non thérapeutique de la peau, comprenant au moins une étape d'application sur celle-ci d'au moins une composition conforme à l'invention.

Selon un autre de ses aspects, la présente invention a également pour objet l'utilisation de particules et/ou au moins d'une dispersion conforme à l'invention pour la fabrication d'une composition destinée à la protection de la peau et/ou des cheveux contre les effets néfastes des rayonnements UV en particulier le rayonnement solaire.

Il est entendu que les compositions conformes à l'invention sont destinées à être appliquées sur toute partie de la peau du corps humain, ou animal, particulièrement toute partie de la peau, y compris les lèvres et le cuir chevelu.

Le calibrage des particules est avantageux dans la mesure où il garantit à celles-ci des distributions de taille et de forme homogènes et garantit aux filtres solaires une stabilité et des performances accrues mesurables en particulier par l'amélioration du SPF.

Elles permettent de façon générale, dans le cadre de la photoprotection, d'optimiser le dépôt à la surface de la peau.

L'utilisation de telles particules d'huiles calibrées et sphériques comprenant un filtre solaire permet en outre

d'introduire des actifs additionnels thermosensibles dans la composition finale, qu'ils soient hydrophiles ou lipophiles.

Un autre avantage qui ressort de leur utilisation est la possibilité d'obtenir des compositions fluides.

Dans le cadre de la présente invention, les termes « gélifié » et « épaissi » peuvent être considérés comme des synonymes du terme « structuré » lorsqu'il s'agit de qualifier les particules huileuses.

Tous les brevets ou demandes de brevet cités ci-après sont incorporés dans la présente demande par référence.

## PARTICULES CALIBREES ET SPHERIQUES

Les particules huileuses calibrées comprenant au moins un filtre solaire et comprenant au moins une huile ou phase huileuse structurée conformes à l'invention possèdent une taille moyenne inférieure ou égale à 20 μm, en particulier inférieure ou égale à 15 μm, et plus particulièrement inférieure ou égale à 12 μm. Avantageusement, la taille moyenne des particules peut varier de 100 nm à 20 μm, de 100 nm à 15 μm, voire de 150 nm à 12 μm.

On entend par "calibrées", au sens de la présente invention, des particules possédant une distribution granulométrique homogène.

Les particules conformes à la présente invention présentant une taille moyenne inférieure à 15 μm et permettent un relargage contrôlé et avantageux du filtre solaire sur la peau et/ou les cheveux.

La distribution granulométrique qualifie la répartition de la taille des particules calibrées autour d'une taille moyenne. La distribution granulométrique peut être caractérisée par un indice de polydispersité ou un coefficient d'uniformité. Plus la valeur de l'indice, ou du coefficient, est faible, plus les tailles des particules se répartissent de manière homogène autour d'une taille moyenne.

Ainsi, pour les particules huileuses calibrées submicroniques, conformes à l'invention, c'est-à-dire de taille moyenne inférieure au micromètre, la distribution granulométrique peut être caractérisée par un indice de polydispersité, noté IP (valeur sans dimension et caractérisant l'étendue de la distribution granulométrique). Cet indice est alors avantageusement inférieur ou égal à 0,35, et de préférence supérieur ou égal à 0,01.

La taille des particules huileuses calibrées, submicroniques, peut être déterminée, par exemple, avec un granulomètre laser fonctionnant sur le principe de la diffusion quasi élastique de la lumière, comme le BI90PLUS® de BROOKHAVEN INSTRUMENT®.

Pour les particules huileuses calibrées de taille moyenne supérieure au micromètre, la distribution granulométrique peut être caractérisée par un coefficient d'uniformité mesuré à l'aide d'un granulomètre à diffraction laser, comme par exemple le MASTER SIZER 2000® de MALVERN. Les particules huileuses calibrées conformes à l'invention dont la taille est supérieure au micromètre, peuvent posséder un coefficient d'uniformité avantageusement inférieur ou égal à 0,45, et de préférence supérieur ou égal à 0,1.

La taille des particules, et l'homogénéité de distribution granulométrique autour d'une taille moyenne sont généralement déterminées par la nature du procédé utilisé lors de leur obtention. Les procédés permettant d'obtenir les particules calibrées et sphériques conformes à l'invention sont décrits par la suite.

Les particules calibrées présentent une forme avantageusement homogène et substantiellement sphérique.

Par "substantiellement sphérique", on entend signifier que les particules sont de forme substantiellement isotrope, c'est-à-dire qu'elles possèdent une morphologie relativement régulière.

On définit ainsi, dans le cadre de la présente invention, un paramètre relatif au facteur de forme des particules comme par exemple l'indice de circularité C, défini comme le rapport de la surface totale A de la particule sur la surface du disque ayant le même périmètre P :

$$C = 4\Pi A/P$$

avec C compris entre 0,9 et 1.

Cette mesure peut avantageusement être effectuée avec un appareil Sysmex FPIA 2100, granulomètre par analyse d'image.

Par ailleurs, outre l'homogénéité de forme et de distribution de taille, les particules calibrées et sphériques conformes à l'invention sont avantageusement homogènes quant à leur structure.

Ainsi, le gel huileux de la particule, obtenu par la structuration d'au moins une huile ou phase huileuse avec au moins un polymère gélifiant, présente avantageusement une structure homogène.

La distribution du filtre solaire est également avantageusement homogène dans l'ensemble de la particule.

**HUILE**

Les particules huileuses calibrées et sphériques comportant un filtre solaire conformes à la présente invention comprennent au moins une huile ou phase huileuse, contenant notamment au moins une huile liquide à température ambiante (20 - 25 °C) et à pression atmosphérique.

On entend désigner par "phase huileuse", au sens de la présente invention, une phase comprenant au moins une huile. Cette huile est avantageusement une huile non volatile. Au sens de la présente invention, on entend par "huile non-volatile", une huile ayant une pression de vapeur inférieure à 0,13 Pa.

La phase huileuse, convenant à la mise en oeuvre des particules conformes à la présente invention peut, par exemple, comprendre au moins une huile choisie parmi une huile végétale, une huile animale, une huile synthétique, minérale et leurs mélanges.

La phase huileuse peut, également, comprendre en outre au moins une huile volatile qui peut requérir des conditions particulières du procédé, notamment sous pression.

Au sens de la présente invention, on entend par "huile volatile", une huile (ou milieu non aqueux) susceptible de s'évaporer au contact de la peau (par exemple aux environs de 33 °C) en moins d'une heure, à température ambiante et à pression atmosphérique. L'huile volatile est une huile cosmétique volatile, liquide à température ambiante, ayant notamment une pression de vapeur non nulle, à température ambiante et pression atmosphérique, en particulier ayant une pression de vapeur allant de 0,13 Pa à 40 000 Pa ($10^{-3}$ à 300 mm Hg), et de préférence allant de 1,3 Pa à 13 000 Pa (0,01 à 100 mm Hg) et préférentiellement allant de 1,3 Pa à 1300 Pa (0,01 à 10 mm Hg).

Les huiles convenant à la mise en oeuvre de l'invention peuvent être d'origine naturelle, végétale ou minérale, ou d'origine synthétique. Elles peuvent être de type hydrocarboné telles que, par exemple, les triglycérides, esters, alcanes, polyoléfines, de type silicone ou de type fluorée, modifiée ou non.

Au sens de la présente invention, on entend par "huile fluorée", une huile comprenant au moins un atome de fluor.

Au sens de la présente invention, on entend par "huile siliconée", une huile comprenant au moins un atome de silicium, et notamment au moins un groupe Si-O.

On entend désigner par "huile hydrocarbonée", une huile contenant principalement des atomes d'hydrogène et de carbone et éventuellement des atomes d'oxygène, d'azote, de soufre et/ou de phosphore.

Selon un mode de réalisation, elles peuvent être utilisées seules ou en mélange, entre elles ou avec d'autres composés tels que définis, par exemple, par la suite.

Avantageusement, les huiles utilisées pour la mise en oeuvre de l'invention sont compatibles avec le polymère gélifiant utilisé pour structurer la phase huileuse.

Les huiles non volatiles peuvent, notamment, être choisies parmi les huiles hydrocarbonées, le cas échéant fluorées et/ou les huiles siliconées non volatiles.

Comme huile hydrocarbonée non volatile, on peut notamment citer :

- les huiles hydrocarbonées d'origine animale, telle que le squalane ;
- les huiles hydrocarbonées d'origine végétale telles que les esters de phytostéaryle, comme l'oléate de phytostéaryle, l'isostéarate de physostéaryle et le glutamate de lauroyl/octyldodécyle/phytostéaryle (AJINOMOTO, ELDEW PS203) ; les triglycérides constitués d'esters d'acides gras et de glycérol dont les acides gras peuvent avoir des longueurs de chaînes variées de $C_4$ à $C_{24}$, ces dernières pouvant être linéaires ou ramifiées, saturées ou insaturées, ces huiles sont notamment des triglycérides héptanoïques ou octanoïques ; les huiles de germe de blé, de tournesol, de pépins de raisin, de sésame, de maïs, d'abricot, de ricin, de karité, d'avocat, d'olive, de soja, d'amande douce, de palme, de colza, de coton, de noisette, de macadamia, de jojoba, de luzerne, de pavot, de potimarron, de courge, de cassis, d'onagre, de millet, d'orge, de quinoa, de seigle, de carthame, de bancoulier, de passiflore, de rosier muscat ; le beurre de karité ; ou encore les triglycérides des acides caprylique/caprique comme ceux vendus par la société STÉARINERIES DUBOIS ou ceux vendus sous les dénominations MIGLYOL 810®, 812® et 818® par la société DYNAMIT NOBEL et leurs mélanges,
- les hydrocarbures linéaires ou ramifiés, d'origine minérale ou synthétique tels que la vaseline, les polydécènes, le polyisobutène hydrogéné tel que le parléam, et leurs mélanges ;
- les éthers de synthèse ayant de 10 à 40 atomes de carbone ;
- les esters de synthèse comme les huiles de formule $R_1COOR_2$ dans laquelle $R_1$ représente le reste d'un acide gras linéaire ou ramifié comportant de 1 à 40 atomes de carbone et $R_2$ représente une chaîne hydrocarbonée notamment ramifiée contenant de 1 à 40 atomes de carbone à condition que $R_1 + R_2$ soit $\geq 10$ ;
- et leurs mélanges.

Les esters peuvent être notamment choisis parmi les esters d'acide gras comme par exemple :

- l'octanoate de cétostéaryle, les esters de l'alcool isopropylique, tels que le myristate d'isopropyle, le palmitate

d'isopropyle, le lauroylsarcosinate d'isopropyle, le palmitate d'éthyle, le palmitate de 2-éthyl-hexyle, le stéarate ou l'isostéarate d'isopropyle, l'isostéarate d'isostéaryle, le stéarate d'octyle, les esters hydroxylés comme le lactacte d'isostéaryle, l'hydroxystéarate d'octyle, l'adipate de diisopropyle, les heptanoates, et notamment l'heptanoate d'isostéaryle, les octanoates, décanoates ou ricinoléates d'alcools ou de polyalcools comme le dioctanoate de propylène glycol, l'octanoate de cétyle, l'octanoate de tridécyle, le 4-diheptanoate d'éthyle 2-hexyle, le diheptanoate de polyéthylène glycol, le diétyl 2-d'hexanoate de propylèneglycol et leurs mélanges, le laurate d'hexyle, les esters de l'acide néopentanoïque comme le néopentanoate d'isodécyle, le néopentanoate d'isotridécyle, le néopentanoate d'isostéaryle, le néopentanoate d'octyldocécyle, les esters de l'acide isononanoïque comme l'isononanoate d'isononyle, l'isononanoate d'isotridécyle, l'isononanoate d'octyle, les esters hydroxylés comme le lactate d'isostéaryle, le malate de di-isostéaryle, le benzoate d'alkyle, les benzoates d'alcools en $C_{12}$ à $C_{15}$, et leurs mélanges ;

- les esters de polyols et les esters du pentaérythritol, comme le tétrahydroxystéarate/tétraisostéarate de dipentaérythritol ;
- les esters de dimères diols et dimères diacides tels que les Lusplan DD-DA5® et DD-DA7®, et leurs mélanges, commercialisés par la société NIPPON FINE CHEMICAL et décrits dans la demande FR 0 302 809 déposée le 6 mars 2003, dont le contenu est incorporé dans la présente demande par référence ;
- les alcools gras liquides à température ambiante à chaîne carbonée ramifiée et/ou insaturée ayant de 12 à 26 atomes de carbone comme le 2-octyldodécanol, l'alcool isostéarylique, l'alcool oléique, le 2-hexyldécanol, le 2-butyloctanol, et le 2-undécylpentadécanol ;
- les acides gras supérieurs liquides tels que l'acide oléique, l'acide linoléique, l'acide linolénique, et leurs mélanges ;
- les carbonates de di-alkyle, les 2 chaînes alkyles pouvant être identiques ou différentes, tel que le dicaprylyl carbonate commercialisé sous la dénomination CETIOL CC®, par COGNIS ; et
- leurs mélanges.

Les huiles de silicone non volatiles utilisables dans la composition selon l'invention peuvent être les polydiméthylsiloxanes (PDMS) non volatiles, comme la siméthicone, les polydiméthylsiloxanes comportant des groupements alkyle ou alcoxy pendants et/ou en bouts de chaîne siliconée, groupements ayant chacun de 2 à 24 atomes de carbone, les silicones phénylées comme les phényltriméthicones, les phényldiméthicones, les phényltriméthylsiloxydiphénylsiloxanes, les diphényldiméthicones, les diphénylméthyldiphényltrisiloxanes, et les 2-phényléthyltriméthylsiloxysilicates, les diméthicones ou phényltriméthicone de viscosité inférieure ou égale à 100 cst, et leurs mélanges.

Les huiles hydrocarbonées volatiles optionnellement présentes peuvent être choisies, notamment, parmi les huiles hydrocarbonées ayant de 8 à 16 atomes de carbone, et notamment les alcanes ramifiés en $C_8$-$C_{16}$ (appelées aussi isoparaffines), comme l'isododécane (encore appelé 2,2,4,4,6-pentaméthylheptane), l'isodécane, l'isohexadécane, et par exemple les huiles vendues sous les noms commerciaux d'ISOPARS® ou de PERMETHYLS®.

Comme huiles volatiles optionnellement présentes, on peut aussi utiliser les silicones volatiles, comme par exemple les huiles de silicones linéaires ou cycliques volatiles, notamment celles ayant une viscosité $\leq$ 8 centistokes ($8 \times 10^{-6}$ $m^2/s$), et ayant notamment de 2 à 10 atomes de silicium, et en particulier de 2 à 7 atomes de silicium. Ces huiles de silicone comportent éventuellement des groupes alkyle ou alkoxy ayant de 1 à 10 atomes de carbone. Comme huile de silicone volatile utilisable dans l'invention, on peut citer notamment les diméthicones de viscosité 5 et 6 cSt comme l'octaméthylcyclotétrasiloxane, le décaméthylcyclopentasiloxane, le dodécaméthylcyclohexasiloxane, l'heptaméthylhexyl-trisiloxane, l'heptaméthyloctyltrisiloxane, l'hexaméthyldisiloxane, l'octaméthyltrisiloxane, le décaméthyltétrasiloxane, le dodécaméthylpentasiloxane, et leurs mélanges.

On peut également utiliser des huiles volatiles fluorées telles que le nonafluorométhoxybutane ou le perfluorométhylcyclopentane, et leurs mélanges.

De manière avantageuse, la phase huileuse présente dans les particules conformes à la présente invention, peut comprendre au moins une huile notamment choisie parmi le squalane, l'isononanoate d'isononyle, le lauroylsarcosinate d'isopropyle, l'octyldodécanol, et leurs mélanges.

La teneur en phase huileuse des particules conformes à l'invention peut varier de 10 à 99 % en poids, en particulier d'au moins 15 à 99 % en poids, plus particulièrement de 20 à 99 % en poids, par rapport au poids total de la particule calibrée selon l'invention.

Selon un mode de réalisation, la phase huileuse, structurée par au moins un polymère gélifiant formant des particules huileuses calibrées conformes à l'invention, possède un point de fusion supérieur ou égal à 40 °C, de préférence inférieur ou égal à 95°C. Selon un mode de réalisation préféré, la même phase huileuse possède un point de fusion pouvant varier entre 50 et 90 °C.

La phase huileuse structurée dont dérivent les particules huileuses calibrées conformes à l'invention possède une viscosité supérieure à 750 Pa.s au cisaillement de 1 $s^{-1}$ à 25 °C, de préférence inférieure ou égale à $1.10^6$ Pa.s.

La viscosité de l'huile ou phase huileuse structurée à l'aide d'au moins un polymère gélifiant, peut être déterminée à l'aide d'un rhéomètre (RFS3 Rhéométrics).

Les mesures sont alors faites à 25 °C, la température étant régulée par effet Peltier.

La géométrie est un cône / plan, cône de 25 mm de diamètre et angle 2°.

On impose un gradient de vitesse de 1 $s^{-1}$, pendant un certain temps d'équilibre, 5 minutes par exemple. La viscosité est donnée en Pa.s, à température et temps donnés.

La viscosité est en réalité mesurée à un stade où les particules ne sont pas encore formées, à savoir au niveau du pré-mélange huile, gélifiant et filtre solaire hydrophobe, comme cela ressort à la lecture du procédé détaillé ci-après.

Le choix de l'huile ou des huiles rentrant dans la formulation de la phase huileuse ainsi que celui du ou des polymère(s) gélifiant(s) peuvent être ajustés, par l'homme du métier, de manière à ce que la phase huileuse structurée, des particules conformes à l'invention réponde aux critères de point de fusion et de viscosité précédemment décrits.

## POLYMÈRE GÉLIFIANT

Les particules huileuses calibrées et sphériques sont avantageusement obtenues à partir d'au moins une phase huileuse structurée par au moins un polymère gélifiant choisi notamment parmi un polymère semi-cristallin, un monoalkyl- ou polyalkyl-ester de polysaccharide, un polyamide, un polyamide siliconé, un copolymère di-bloc, tri-bloc, multi-bloc et/ou radial-bloc, et leurs mélanges.

Par "polymère", on entend au sens de la présente invention désigner des composés comportant au moins deux motifs de répétition, de préférence au moins trois motifs de répétition et notamment au moins 10 motifs de répétition.

La teneur en polymère(s) gélifiant(s), dans les particules conformes à l'invention, peut varier de 1 à 80 % en poids, voire de 1 à 60 % en poids par rapport au poids total de la particule.

Avantageusement, le rapport pondéral entre le polymère gélifiant et la phase huileuse de la particule selon l'invention peut varier de 0.01 à 4, voire de 0,05 à 2, et en particulier de 0,1 à 1.

### *Polymère semi-cristallin*

Par "polymère semi-cristallin", on entend au sens de l'invention, des polymères comportant une partie cristallisable, chaîne pendante ou séquence dans le squelette, et une partie amorphe dans le squelette et présentant une température de changement de phase réversible du premier ordre, en particulier de fusion (transition solide-liquide). Lorsque la partie cristallisable est une séquence du squelette polymérique, cette séquence cristallisable est de nature chimique différente de celle des séquences amorphes ; le polymère semi-cristallin est dans ce cas un polymère séquencé par exemple du type dibloc, tribloc ou multibloc.

Les polymères semi-cristallins utilisables pour la mise en oeuvre de la présente invention sont solides à température ambiante et présentent de préférence une température de fusion (ou gélification) inférieure à 80 °C.

Ils comportent :

a) un squelette polymérique, et

b) au moins une chaîne organique latérale cristallisable et/ou une séquence organique cristallisable faisant partie du squelette dudit polymère, ledit polymère ayant une masse moléculaire moyenne en nombre supérieure ou égale à 2 000.

De façon avantageuse, le ou les polymères semi-cristallins de la composition de l'invention ont une masse moléculaire moyenne en nombre Mn supérieure ou égale à 2000, allant par exemple de 2 000 à 800 000, notamment de 3 000 à 500 000.

Selon un mode de réalisation, les polymères semi-cristallin utilisables dans le cadre de l'invention présentent une température de fusion (ou point de fusion) pF inférieure à 70 °C, notamment inférieure à 50 °C. Le polymère semi-cristallin a avantageusement une température de fusion pF comprise dans l'intervalle allant de 40 °C à moins de 80 °C. En réalité, le polymère semi-cristallin peut être un mélange de polymères semi-cristallin. Dans ce cas, c'est le mélange qui présente une température de fusion PF comrpise dans ledit intervalle. Autrement dit, le mélange peut comprendre un polymère semi-cristallin présentant un point de fusion en dehors de cet intervalle, pour autant que le mélange, quant à lui, présente une température de point de fusion dans ledit intervalle. La température de fusion peut être mesurée notamment par toute méthode connue et en particulier avec un calorimètre à balayage différentiel (D.S.C).

Selon une variante de réalisation, les séquences ou chaînes cristallisables des polymères semi-cristallins représentent au moins 30 %, voire au moins 40 % du poids total de chaque polymère. Les polymères semi-cristallins de l'invention à séquences cristallisables peuvent être des polymères séquencés ou multiséquencés. Ils peuvent être obtenus par polymérisation de monomères à doubles liaisons réactives (liaisons éthyléniques) ou par polycondensation. Lorsque les polymères de l'invention sont des polymères à chaînes latérales cristallisables, ceux-ci sont avantageusement sous forme aléatoire ou statistique.

Les polymères semi-cristallins utilisables dans l'invention sont, par exemple :

1. les copolymères séquencés de polyoléfines à cristallisation contrôlée, dont les monomères sont décrits dans le document EP-A-951897 ;
2. les polycondensats et notamment les polycondensats polyesters, aliphatiques ou aromatiques, et les copolyesters aliphatiques/aromatiques ;
3. les homo- ou co-polymères portant au moins une chaîne latérale cristallisable et les homo- ou co-polymères portant dans le squelette au moins une séquence cristallisable, comme ceux décrits dans le brevet US-A-5,156,911 ;
4. les homo- ou co-polymères portant au moins une chaîne latérale cristallisable à groupement(s) fluoré(s), tels que décrits dans la demande WO-A-01/19333 ;
5. et leurs mélanges.

Dans les deux derniers cas (3 et 4), la ou les chaînes latérales ou séquences cristallisables sont hydrophobes.
Les polymères cristallins à chaînes latérales cristallisables, ou portant dans le squelette au moins une séquence cristallisable convenant à la mise en oeuvre de l'invention, sont par exemple, décrits ci-dessous.

*A) Polymères semi-cristallins à chaînes latérales cristallisables*

On peut citer notamment les polymères définis dans les documents US 5156911 et WO-A-01/19333. Ce sont des homopolymères ou copolymères comportant de 50 à 100 % en poids de motifs résultant de la polymérisation d'un ou de plusieurs monomères porteurs de chaîne(s) latérale(s) hydrophobe(s) cristallisable(s).
Ces homo- ou co-polymères sont de toute nature du moment qu'ils présentent les conditions indiquées ci-après avec, en particulier, la caractéristique d'être solubles ou dispersables dans la phase huileuse par chauffage au-dessus de leur température de fusion pF (ou gélification). Ils peuvent résulter :

- de la polymérisation notamment radicalaire d'un ou plusieurs monomères à double(s) liaison(s) réactive(s) ou éthyléniques vis-à-vis d'une polymérisation, à savoir à groupe vinylique, (méth)acrylique ou allylique ;
- de la polycondensation d'un ou plusieurs monomères porteurs de groupes coréactifs (acide carboxylique ou sulfonique, alcool, amine ou isocyanate), comme par exemple les polyesters, les polyuréthanes, les polyéthers, les polyurées, les polyamides.

D'une façon générale les motifs (chaînes ou séquences) cristallisables des polymères semi-cristallins utilisables dans le cadre de l'invention, proviennent de monomère(s) à séquence(s) ou chaîne(s) cristallisable(s), utilisé(s) pour la fabrication des polymères semi-cristallins. Ces polymères sont choisis, notamment, parmi les homopolymères et copolymères résultant de la polymérisation d'au moins un monomère à chaîne(s) cristallisable(s) qui peut être représenté par la formule X :

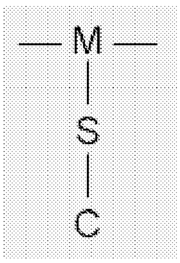

dans laquelle M représente un atome du squelette polymérique, S représente un espaceur et C représente un groupe cristallisable.
Les chaînes "-S-C" cristallisables peuvent être aliphatiques ou aromatiques, éventuellement fluorées ou perfluorées. "S" représente notamment un groupe $(CH_2)n$ ou $(CH_2CH_2O)n$ ou $(CH_2O)$, linéaire ou ramifié ou cyclique, n étant un nombre entier allant de 0 à 22. De préférence "S" est un groupe linéaire. De préférence, "S" et "C" sont différents.
Lorsque les chaînes cristallisables sont des chaînes aliphatiques (alkyle), elles comportent au moins 11 atomes de carbone et au plus 40 atomes de carbone et mieux au plus 24 atomes de carbone. Il s'agit notamment de chaînes alkyle possédant au moins 12 atomes de carbone, et par exemple, il peut s'agir de chaînes alkyle comportant de 14 à 24 atomes de carbone ($C_{14}$-$C_{24}$). Il peut s'agir de chaînes alkyle hydrocarbonées (atomes de carbone et d'hydrogène) ou chaînes alkyle fluorées ou perfluorées (atomes de carbone, atomes de fluor et éventuellement atomes d'hydrogène). Lorsqu'il s'agit de chaînes alkyle fluorées ou perfluorées, elles comportent au moins 11 atomes de carbone dont au moins 6 atomes de carbone sont fluorés.
Comme exemples de polymères ou copolymères semi-cristallins à chaîne(s) cristallisable(s), on peut citer ceux

résultant de la polymérisation d'au moins un monomère à chaîne cristallisable choisi parmi les (méth)acrylates d'alkyle saturés en $C_{14}$-$C_{24}$ ($C_{14}$-$C_{24}$ signifie que le groupe alkyle comporte de 14 à 24 atomes de carbone) ; les (méth)acrylates de perfluoroalkyle en $C_{11}$-$C_{15}$ (groupe alkyle avec 11 à 15 atomes de carbone) ; les N-alkyl(méth)acrylamides en $C_{14}$ à $C_{24}$ avec ou sans atome de fluor ; les esters vinyliques à chaînes alkyle ou perfluoroalkyle en $C_{14}$ à $C_{24}$, avec une chaîne perfluoroalkyle comportant au moins 6 atomes de fluor ; les éthers vinyliques à chaînes alkyle ou perfluoroalkyle en $C_{14}$ à $C_{24}$, avec une chaîne perfluoroalkyle comportant au moins 6 atomes de fluor ; les alpha-oléfines en $C_{14}$ à $C_{24}$, comme par exemple l'octadécène ; les para-alkyl styrènes en $C_{14}$ à $C_{24}$, leurs mélanges.

Par "alkyle", on entend au sens de l'invention un groupement saturé notamment, comportant de 8 à 24 atomes de carbone ($C_8$ à $C_{24}$), sauf mention exprès.

Lorsque les polymères résultent d'une polycondensation, les chaînes cristallisables hydrocarbonées et/ou fluorées telles que définies ci-dessus, sont portées par un monomère qui peut être un diacide, un diol, une diamine, un diisocyanate.

Lorsque les polymères utilisés dans la composition de l'invention sont des copolymères, ils contiennent, en plus, de 0 à 50 % de groupes Y ou Z résultant de la copolymérisation :

$\alpha$) avec Y qui est un monomère polaire ou non polaire ou un mélange des deux :

- lorsque Y est un monomère polaire, c'est soit un monomère porteur de groupes polyoxyalkylénés (notamment oxyéthylénés et/ou oxypropylénés), un (méth)acrylate d'hydroxyalkyle comme l'acrylate d'hydroxyéthyle, le (méth)acrylamide, un N-alkyl(méth)acrylamide, un N,N-dialkyl(méth)acrylamide comme par exemple le N,N-diisopropylacrylamide ou la N-vinyl-pyrrolidone (NVP), le N-vinyl-caprolactame, soit un monomère porteur d'au moins un groupe acide carboxylique comme les acides (méth)acryliques, crotonique, itaconique, maléique, fumarique ou porteur d'un groupe anhydride d'acide carboxyliquecomme l'anhydre maléique, et leurs mélanges.
- lorsque Y est un monomère non polaire, il peut être un ester du type (méth)acrylate d'alkyle linéaire ramifié ou cyclique, un ester vinylique, un alkyl vinyl éther, une alpha-oléfine, le styrène ou le styrène substitué par un groupe alkyle comportant de 1 à 10 atomes de carbone ($C_1$ à $C_{10}$), comme l'$\alpha$-méthylstyrène, un macromonomère du type polyorganosiloxane à insaturation vinylique.

$\beta$) avec Z qui est un monomère polaire ou un mélange de monomères polaires, Z ayant la même définition que le "Y polaire" défini ci-dessus.

De préférence, les polymères semi-cristallins à chaîne latérale cristallisable sont choisis parmi les homopolymères d'alkyl(méth)acrylate ou d'alkyl(méth)acrylamide avec un groupe alkyle tel que défini ci-dessus, et notamment en $C_{14}$-$C_{24}$ les copolymères de ces monomères avec un monomère hydrophile de préférence de nature différente de l'acide (méth)acrylique ; et leurs mélanges. Il peut s'agir, par exemple, comme copolymères, des copolymères d'alkyl (méth)acrylate ou d'alkyl (méth)acrylamide avec un groupe alkyle en $C_{14}$ à $C_{24}$, avec la N-vinylpyrrolidone, l'hydroxyéthyl (méth)acrylate ; ou leurs mélanges.

*B) Les polymères portant dans le squelette au moins une séquence cristallisable*

Il s'agit encore de polymères solubles ou dispersables dans l'huile ou phase huileuse par chauffage au-dessus de leur point de fusion pF. Ces polymères sont notamment des copolymères séquencés constitués d'au moins 2 séquences de nature chimique différente dont l'une est cristallisable.

A titre de polymères portant dans le squelette au moins une séquence cristallisable convenant à la mise en oeuvre de l'invention, on peut mentionner:

1. Les polymères définis dans le document US-A-5,156,911 ;
2. Les copolymères séquencés d'oléfine ou de cyclooléfine à chaîne cristallisable comme ceux issus de la polymérisation séquencée de :

- cyclobutène, cyclohexène, cyclooctène, norbornène (c'est-à-dire bicyclo(2,2,1)heptène 2), 5-méthylnorbornène, 5-éthylnorbornène, 5,6-diméthylnorbomène, 5,5,6-triméthyl norbornène, 5-éthylidène-norbornène, 5-phénylnorbornène, 5-benzylnorbornène, 5-vinylnorbornène, 1,4,5,8-diméthano-1,2,3,4,4a,5,8a-octahydronaphtalène, dicyclopentadiène, et leurs mélanges ;
- avec l'éthylène, le propylène, le 1-butène, le 3-méthyl-1-butène, le 1-hexène, le 4-méthyl-1-pentène, le 1-octène, le 1-décène, le 1-éicosène ou leurs mélanges. Ces copolymères séquencés peuvent être en particulier les copolymères (éthylène/norbornène) blocs et les terpolymères blocs (éthylène/propylène/ éthylidènenorbomène).

On peut aussi utiliser ceux résultants de la copolymérisation séquencée d'au moins 2 α-oléfines en $C_2$-$C_{16}$ et mieux en $C_2$-$C_{12}$, tels que ceux cités précédemment et en particulier les bipolymères séquencés d'éthylène et d'1-octène.
3. Les copolymères présentant au moins une séquence cristallisable, le reste du copolymère étant amorphe (à température ambiante). Ces copolymères peuvent, en outre, présenter deux séquences cristallisables de nature chimique différente. Les copolymères préférés sont ceux qui possèdent à la fois à température ambiante, une séquence cristallisable et une séquence amorphe à la fois hydrophobe et lipophile réparties séquentiellement ; on peut citer par exemple les polymères possédant une des séquences cristallisables et une des séquences amorphes suivantes :

- Séquence cristallisable par nature : a) polyester comme les poly(alkylène téréphtalate), b) polyoléfine comme les polyéthylènes ou polypropylènes.
- Séquence amorphe et lipophile comme les polyoléfines ou copoly(oléfine)s amorphes telles que le poly(isobutylène), le polybutadiène hydrogéné, le poly(isoprène) hydrogéné.

Comme exemple de tels copolymères à séquence cristallisable et à séquence amorphe, on peut citer :

α) les copolymères séquencés poly(Σ-caprolactone)-b-poly(butadiène), utilisés de préférence hydrogénés, tels que ceux décrits dans l'article "Melting behavior of poly(Σ -caprolactone)-block-polybutadiène copolymers" de S. Nojima, Macromolécules, 32, 3727-3734 (1999).
β) les copolymères séquencés poly(butylènetéréphtalate)-b-poly(isoprène) hydrogénés séquencés ou multiséquencés, cités dans l'article "Study of morphological and mechanical properties of PP/PBT" de B. Boutevin et al., Polymer Bulletin, 34, 117-123 (1995).
γ) les copolymères séquencés poly(éthylène)-b-copoly(éthylène/propylène) cités dans les articles "Morphology of semi-crystalline block copolymers of ethylene- (ethylene-alt-propylene)" de P. Rangarajan et al., Macromolecules, 26, 4640-4645 (1993), et "Polymer agregates with crystalline cores : the system poly(ethylene)- poly(ethylene-propylene)" P. Richter et al., Macromolécules, 30, 1053-1068 25 (1997).
δ) les copolymères séquencés poly(éthylène)-b-poly(éthyléthylène) cités dans l'article général "Cristallization in block copolymers" de I.W. Hamley, Advances in Polymer Science, vol 148, 113-137 (1999).

Les polymères semi-cristallins utilisables dans le cadre de l'invention peuvent être non réticulés ou réticulés en partie, du moment que le taux de réticulation ne gêne pas leur dissolution ou dispersion dans la phase huileuse liquide par chauffage au dessus de leur température de fusion. Il peut s'agir alors d'une réticulation chimique, par réaction avec un monomère multifonctionnel lors de la polymérisation. Il peut aussi s'agir d'une réticulation physique qui peut alors être due soit à l'établissement de liaisons type hydrogène ou dipolaire entre des groupes portés par le polymère, comme par exemple les interactions dipolaires entre ionomères carboxylates, ces interactions étant en faible quantité et portées par le squelette du polymère ; soit à une séparation de phase entre les séquences cristallisables et les séquences amorphes portées par le polymère.
De préférence, les polymères semi-cristallins convenant à l'invention sont non réticulés.
A titre d'exemple particulier de polymère semi-cristallin utilisable dans la composition selon l'invention, on peut citer les produits INTELIMER® de la société LANDEC décrits dans la brochure "INTELIMER® POLYMERS". Ces polymères sont sous forme solide à température ambiante (25 °C). Ils sont porteurs de chaînes latérales cristallisables et présentent le monomère tel que défini dans la formule X précédente. On peut citer notamment le "LANDEC IP22®", ayant une température de fusion pF de 56 °C, qui est un produit visqueux à température ambiante, imperméable, non-collant.
On peut aussi utiliser le polymère "STRUCTURE O" commercialisé par la société NATIONAL STARCH, tel que celui décrit dans le document US-A-5,736,125, de pF 44 °C, ainsi que les polymères semi-cristallins à chaînes pendantes cristallisables comportant des groupements fluorés tels que décrits dans les exemples 1, 4, 6, 7 et 8 du document WO-A-01/19333.
On peut encore utiliser les polymères semi-cristallins obtenus par copolymérisation d'acrylate de stéaryle et d'acide acrylique ou de NVP ou par copolymérisation de l'acrylate de béhényle et de l'acide acrylique ou de NVP, tels que décrits dans le document US-A-5,519,063 ou EP-A- 0550745.
Selon une variante particulière de réalisation, les polymères semi-cristallins convenant à la mise en oeuvre de la présente invention sont notamment les acrylates alkylés, parmi lesquels on peut mentionner les copolymères de LANDEC :

- DORESCO IPA 13-1® : poly acrylate de stéaryle, pf de 49 °C et PM de 145000;
- DORESCO IPA 13-3® : poly acrylate/ acide méthacrylique, pf de 65 °C et PM de 114000;
- DORESCO IPA 13-4® : poly acrylate/ vinyl pirrolidone, pf de 44 °C et PM de 387000;

- DORESCO IPA13-5® : poly acrylate / methacrylate d'hydroxyethyle, pf de 47 °C et PM de 397600;
- DORESCO IPA 13-6® : poly acrylate de béhényle, pf de 66 °C.

*Les polyamides*

Les polyamides avantageusement utilisables dans la préparation des particules selon la présente invention sont, notamment, ceux décrits dans le document US-A-5 783 657 de la Société UNION CAMP.
Les polyamides convenant à la mise en oeuvre de l'invention satisfont notamment à la formule suivante :

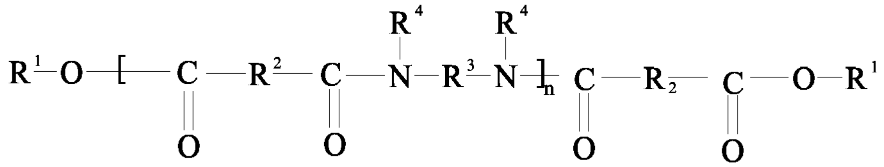

dans laquelle :

- n désigne un nombre entier de motifs amide tel que le nombre de groupes ester représente de 10 % à 50 % du nombre total des groupes ester et amide ;
- $R^1$ est à chaque occurrence, indépendamment, un groupe alkyle ou alcényle ayant au moins 4 atomes de carbone et notamment de 4 à 24 atomes de carbone ;
- $R^2$ représente à chaque occurrence, indépendamment, un groupe hydrocarboné en $C_4$ à $C_{55}$ à condition que 50 % au moins des groupes $R^2$ représentent un groupe hydrocarboné en $C_{30}$ à $C_{55}$;
- $R^3$ représente à chaque occurrence, indépendamment, un groupe organique pourvu d'au moins 2 atomes de carbone, d'atomes d'hydrogène et optionnellement d'un ou plusieurs atomes d'oxygène ou d'azote ; et
- $R^4$ représente à chaque occurrence, indépendamment, un atome d'hydrogène, un groupe alkyle en $C_1$ à $C_{10}$ ou une liaison directe à $R^3$ ou à un autre $R^4$ de sorte que l'atome d'azote auquel sont liés à la fois $R^3$ et $R^4$ fasse partie d'une structure hétérocyclique définie par $R^4$-N-$R^3$, avec au moins 50 % des $R^4$ représentant un atome d'hydrogène.

Selon une variante de réalisation, les groupes esters de ces polyamides représentent de 15 à 40 % du nombre total des groupes ester et amide et au mieux de 20 à 35 %. De plus, n représente avantageusement un nombre entier allant de 1 à 10, et mieux de 1 à 5.
Notamment, $R^1$ est un groupe alkyle en $C_{12}$ à $C_{22}$, voire en $C_{16}$ à $C_{22}$. $R^2$ peut être notamment un groupe hydrocarboné (alkylène) en $C_{10}$ à $C_{42}$. Notamment, 50 % au moins, et mieux 75 % au moins des $R^2$ peuvent être des groupes ayant de 30 à 42 atomes de carbone. Les autres $R^2$ sont des groupes hydrogénés en $C_4$ à $C_{19}$, et en particulier en $C_4$ à $C_{12}$. $R^3$ peut représenter un groupe hydrocarboné en $C_2$ à $C_{36}$ ou un groupe polyoxyalkyléné et $R^4$ représente un atome d'hydrogène. Notamment, $R^3$ peut représenter un groupe hydrocarboné en $C_2$ à $C_{12}$. Les groupes hydrocarbonés peuvent être des groupes linéaires, cycliques ou ramifiés, saturés ou insaturés. Par ailleurs, les groupes alkyle et alkylène peuvent être des groupes linéaires ou ramifiés, saturés ou non.
A titre d'exemple de polyamide structurant utilisable dans l'invention, on peut encore citer les résines polyamides résultant de la condensation d'un acide di-carboxylique aliphatique et d'un diamine (incluant les composés ayant plus de deux groupes carbonyle et deux groupes amine), les groupes carbonyle et amine de motifs unitaires adjacents étant condensés par une liaison amide. Ces résines polyamides sont notamment celles commercialisées sous la marque VERSAMID par les sociétés GENERAL MILLS, Inc. et HENKEL CORP., sous la marque ONAMID notamment ONAMID S ou C. Ces résines ont une masse moléculaire moyenne en poids allant de 6000 à 9000. Les documents US-A-3645705 et US-A-3148125 décrivent ces résines. Selon une variante de réalisation, on utilise les VERSAMID 930 ou 744.
On peut aussi utiliser les polyamides vendus ou fabriqués par la société ARIZONA sous les références UNI-REZ (2658, 2931, 2970, 2621,2613, 2624, 2665, 1554, 2623, 2662) et le produit vendu sous la référence MACROMELT 6212® par la société HENKEL. Le brevet US-A-5500209 décrit ce type de polymères.
A titre d'exemple de polyamides structurant utilisables dans la composition selon l'invention, on peut encore citer les produits commerciaux vendus ou fabriqués par la société ARIZONA CHEMICAL sous les noms UNICLEAR 80® et UNICLEAR 100®. Ils sont vendus respectivement sous forme de gel à 80 % (en matière active) et à 100 % (en matière active) dans une huile minérale. Ils ont un point de ramollissement de 88 à 105 °C. Ces produits commerciaux sont un mélange de copolymère d'un diacide en $C_{36}$ condensé sur l'éthylène diamine, de masse moléculaire moyenne d'environ 6000. Les groupes ester terminaux résultent de l'estérification des terminaisons d'acide restantes par l'alcool cétylique, stéarylique ou leurs mélanges (appelés aussi alcool cétylstéarylique).

Les polyamides structurant ont avantageusement une température de ramollissement supérieure à 60 °C et pouvant aller jusqu'à 190 °C. De préférence, ils présentent une température de ramollissement inférieure à 150 °C allant de 70 à 130 °C et mieux de 80 °C à 105 °C.

La structuration de la phase huileuse peut être obtenue à l'aide d'un ou plusieurs polyamides définis ci-dessus. En général, ces polyamides se présentent sous forme de mélanges, ces mélanges pouvant en outre contenir un produit de synthèse correspondant à un polyamide tel que défini ci-dessus avec n valant 0, c'est-à-dire un diester.

Les polyamides utilisés dans la présente demande présentent du fait de leur chaîne grasse, une bonne solubilité dans la phase huileuse et donc conduisent à des compositions macroscopiquement homogènes même avec un taux élevé de polymère.

A titre d'exemple de polyamide convenant à la mise en oeuvre de la présente invention, on peut mentionner le copolymère d'éthylène diamide/stéaryle dimèrdilinoléate, commercialisé sous la référence UNICLEAR 100® VG, par la société ARIZONA CHEMICAL.

*Polyamides siliconés*

Les polymères (homopolymères ou copolymères) de type polyamide, convenant à la mise en oeuvre de l'invention, possèdent une masse moléculaire moyenne comprise dans l'intervalle allant de 500 à 500 000, et possèdent au moins un groupe comprenant :

- au moins un groupe polyorganosiloxane, comportant de 1 à 1000 unités organosiloxane, dans la chaîne du groupe ou sous forme de greffon, et
- au moins deux groupes capables d'établir des interactions hydrogènes choisis parmi les groupes ester, amide, sulfonamide, carbamate, thiocarabamate, urée, thiourée, oxamido, guanidino, biguanidino, et leurs combinaisons, à condition qu'au moins un de ces groupes soit différent d'un groupe ester,

  le polymère étant solide à température ambiante et soluble dans la phase huileuse à une température variant de 25 à 150 °C. En particulier, le polymère est soluble dans la phase huileuse à une température variant de 41 à 120 °C.

Les polymères convenant à la mise en oeuvre de l'invention, et utilisés comme agent gélifiant de l'huile, peuvent appartenir aux deux familles suivantes :

- des polyorganosiloxanes comprenant au moins deux groupes capables d'établir des interactions hydrogènes, ces deux groupes étant disposés dans la chaîne du polymère, et/ou,
- des polyorganosiloxanes comprenant au moins deux groupes capables d'établir des interactions hydrogènes, ces deux groupes étant disposés sur des greffons ou ramifications.

Les polymères comportant deux groupes capables d'établir des interactions hydrogène dans la chaîne du polymère peuvent être des polymères comprenant au moins un motif répondant à la première formule suivante :

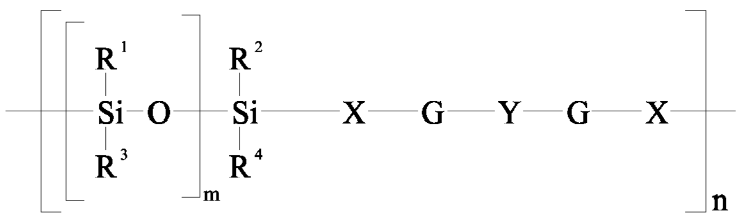

dans laquelle :

1. $R^1$, $R^2$, $R^3$ et $R^4$, identiques ou différents, représentent un groupe choisi parmi :

- les groupes hydrocarbonés, linéaires, ramifiés ou cycliques, en $C_1$ à $C_{40}$, saturés ou insaturés, pouvant contenir dans leur chaîne un ou plusieurs atomes d'oxygène, de soufre et/ou d'azote, et pouvant être substitués en partie ou totalement par des atomes de fluor,
- les groupes aryles en $C_6$ à $C_{10}$, éventuellement substitués par un ou plusieurs groupes alkyle en $C_1$ à $C_4$,
- les chaînes polyorganosiloxanes contenant ou non un ou plusieurs atomes d'oxygène, de soufre et/ou d'azote ;

2. les X, identiques ou différents, représentent un groupe alkylène di-yle, linéaire ou ramifié en $C_1$ à $C_{30}$, pouvant contenir dans sa chaîne un ou plusieurs atomes d'oxygène et/ou d'azote ;
3. Y est un groupe divalent alkylène linéaire ou ramifié, arylène, cycloalkylène, alkylarylène ou arylalkylène, saturé ou insaturé, en $C_1$ à $C_{50}$, pouvant comporter un ou plusieurs atomes d'oxygène, de soufre et/ou d'azote, et/ou porter comme substituant l'un des atomes ou groupes d'atomes suivants : fluor, hydroxy, cycloalkyle en $C_3$ à $C_8$, alkyle en $C_1$ à $C_{40}$, aryle en $C_5$ à $C_{10}$, phényle éventuellement substitué par 1 à 3 groupes alkyle en $C_1$ à $C_3$, hydroxyalkyle en $C_1$ à $C_3$ et amino alkyle en $C_1$ à $C_6$, ou
4. Y représente un groupe répondant à la formule :

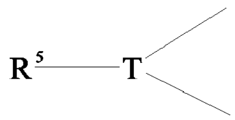

dans laquelle :

- T représente un groupe hydrocarboné trivalent ou tétravalent, linéaire ou ramifié, saturé ou insaturé, en $C_3$ à $C_{24}$ éventuellement substitué par une chaîne polyorganosiloxane, et pouvant contenir un ou plusieurs atomes choisis parmi O, N et S, ou T représente un atome trivalent choisi parmi N, P et Al, et
- $R^5$ représente un groupe alkyle en $C_1$ à $C_{50}$, linéaire ou ramifié, ou une chaîne polyorganosiloxane, pouvant comporter un ou plusieurs groupes ester, amide, uréthane, thiocarbamate, urée, thiourée et/ou sulfonamide qui peut être lié ou non à une autre chaîne du polymère,

■ les G, identiques ou différents, représentent les groupes divalents choisis parmi :

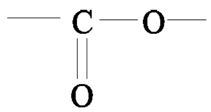 ; 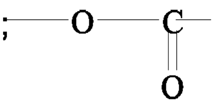 ; 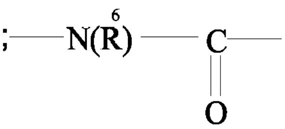 ;

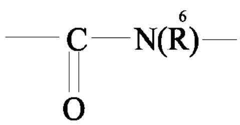 ; 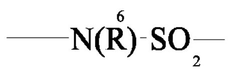 ; 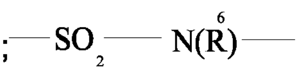 ;

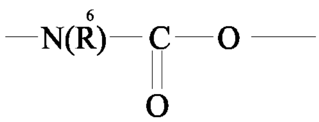 ; 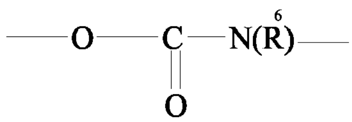 ; 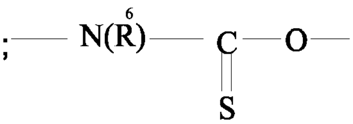 ;

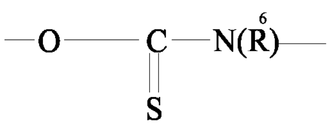 ; 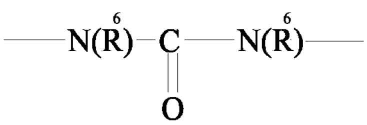

et

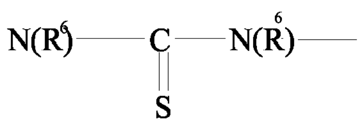

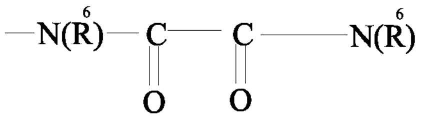 ; 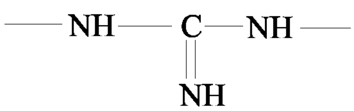 ;

et

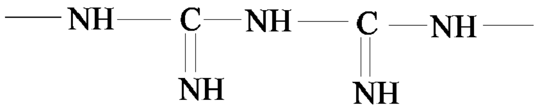

où $R^6$ représente un atome d'hydrogène ou un groupe alkyle, linéaire ou ramifié, en $C_1$ à $C_{20}$.

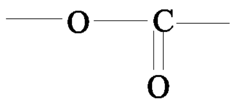 et 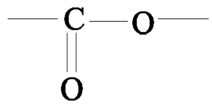 ;

5. n est un nombre entier allant de 2 à 500, en particulier de 2 à 200, et m est un nombre entier allant de 1 à 1000, en particulier de 1 à 700 et mieux encore de 6 à 200.

Selon une variante de réalisation, 80 % des $R^1$, $R^2$, $R^3$ et $R^4$, du polymère peuvent être choisis notamment parmi les groupes méthyle, éthyle, phényle et 3,3,3-trifluoropropyle.

Selon une autre variante de réalisation, Y peut représenter divers groupes divalents, comportant éventuellement de plus une ou deux valences libres pour établir des liaisons avec d'autres motifs du polymère ou copolymère. Notamment, Y peut représenter un groupe choisi parmi :

a) les groupes alkylène linéaires en $C_1$ à $C_{20}$, notamment en $C_1$ à $C_{10}$;
b) les groupes alkylène ramifiés pouvant comporter des cycles et des insaturations non conjuguées, en $C_{30}$ à $C_{56}$ ;
c) les groupes cycloalkylène en $C_5$-$C_6$ ;
d) les groupes phénylène éventuellement substitués par un ou plusieurs groupes alkyle en $C_1$ à $C_{40}$ ;
e) les groupes alkylène en $C_1$ à $C_{20}$, comportant de 1 à 5 groupes amides;
f) les groupes alkylène en $C_1$ à $C_{20}$, comportant un ou plusieurs substituants, choisis parmi les groupes hydroxyle, cycloalcane en $C_3$ à $C_8$, hydroxyalkyle en $C_1$ à $C_3$ et alkylamines en $C_1$ à $C_6$;
g) les chaînes polyorganosiloxane de formule:

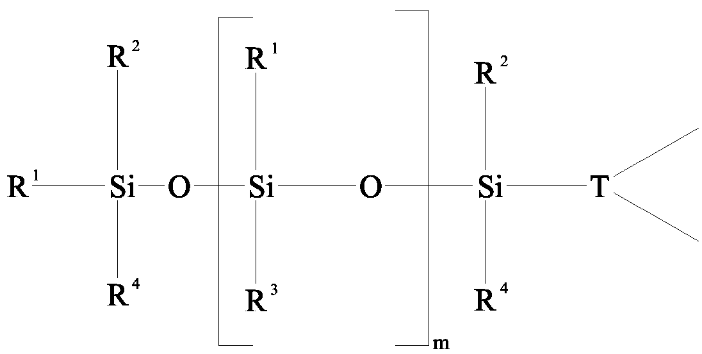

dans laquelle $R^1$, $R^2$, $R^3$ et $R^4$, T et m sont tels que définis ci-dessus; et
h) les chaînes polyorganosiloxanes de formule :

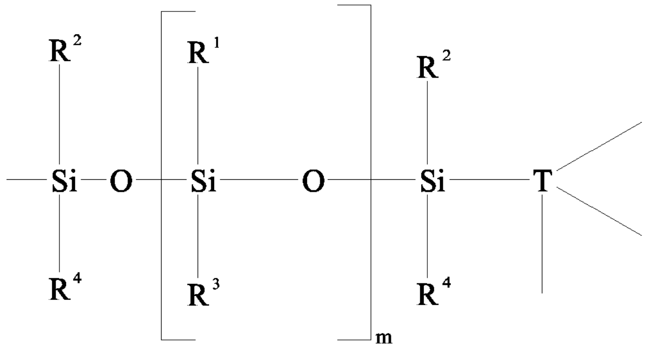

Les polyorganosiloxanes de la seconde famille peuvent être des polymères comprenant au moins un motif répondant à la seconde formule suivante :

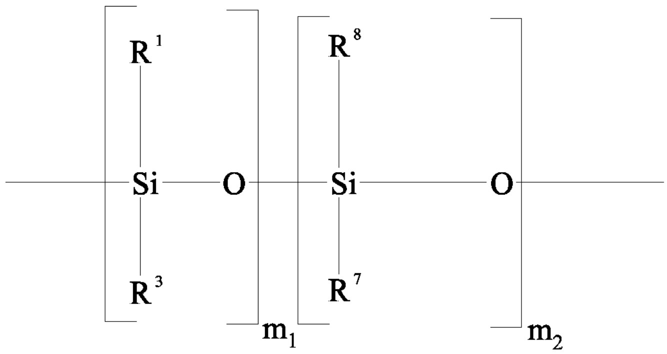

(II)

dans laquelle :

- $R^1$ et $R^3$, identiques ou différents, sont tels que définis ci-dessus pour la formule précédente ;
- $R^7$ représente un groupe tel que défini ci-dessus pour $R^1$ et $R^3$, ou représente le groupe de formule -X-G-$R^9$ dans laquelle X et G sont tels que définis ci-dessus pour la formule précédente et $R^9$ représente un atome d'hydrogène ou un groupe hydrocarboné, linéaire, ramifié ou cyclique, saturé ou insaturé, en $C_1$ à $C_{50}$ comportant éventuellement dans sa chaîne un ou plusieurs atomes choisis parmi O, S et N, éventuellement substitué par un ou plusieurs atomes de fluor et/ou un ou plusieurs groupes hydroxyle, ou un groupe phényle éventuellement substitué par un ou plusieurs groupes alkyle en $C_1$ à $C_4$;
- $R^8$ représente le groupe de formule -X-G-$R^9$ dans laquelle X, G et $R^9$ sont tels que définis ci-dessus ;
- $m_1$ est un nombre entier allant de 1 à 998 ; et
- $m_2$ est un nombre entier allant de 2 à 500.

Selon l'invention, le polyamide siliconé utilisé comme agent gélifiant, peut être un homopolymère, c'est-à-dire un polymère comportant plusieurs motifs identiques, en particulier des motifs selon les formules définies précédemment

Selon l'invention, on peut aussi utiliser un polyamide siliconé constitué par un copolymère comportant plusieurs motifs selon la première formule précédente différents, c'est-à-dire un polymère dans lequel l'un au moins des $R^1$, $R^2$, $R^3$, $R^4$, X, G, Y, m et n est différent dans l'un des motifs. Le copolymère peut être aussi formé de plusieurs motifs selon la seconde formule précédente, dans lequel l'un au moins des $R^1$, $R^3$, $R^7$, $R^8$, $m_1$ et $m_2$ est différent dans l'un au moins des motifs.

On peut encore utiliser un copolymère comportant au moins un motif selon la première formule et au moins un motif selon la seconde formule, les motifs selon la première formule et les motifs selon la seconde formule pouvant être identiques ou différents les uns des autres.

Selon une variante de l'invention, on peut encore utiliser un polyamide siliconé de type copolymère comprenant de plus au moins un motif hydrocarboné comportant deux groupes capables d'établir des interactions hydrogènes choisis parmi les groupes ester, amide, sulfonamide, carbamate, thiocarbamate, urée, thiourée et leurs combinaisons. Ces copolymères peuvent être des copolymères blocs, des copolymères séquencés ou des copolymères greffés.

Selon une variante de réalisation, les groupes capables d'établir des interactions hydrogènes sont des groupes amides de formule -C(O)NH- et -HN-C(O)-. Dans ce cas, l'agent gélifiant peut être, par exemple, un polymère comprenant au moins un motif selon la troisième ou la quatrième formule suivante :

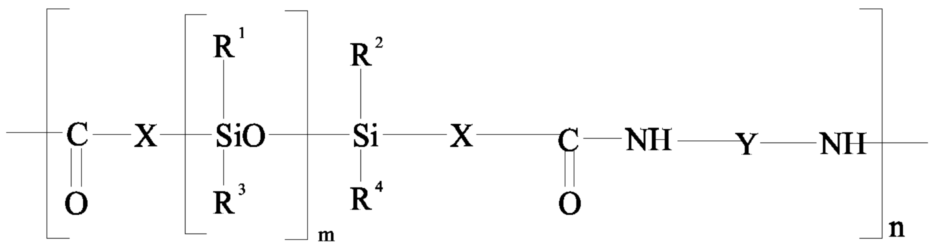

ou

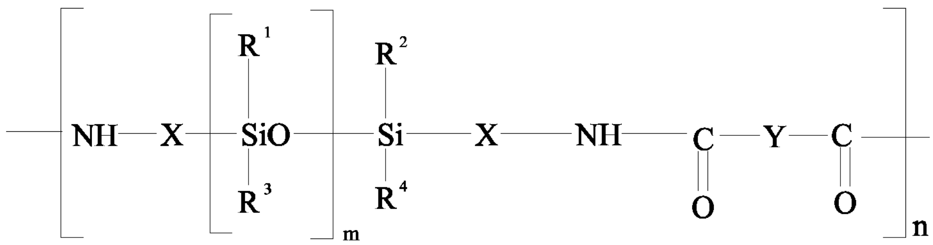

dans lesquelles $R^1$, $R^2$, $R^3$, $R^4$, X, Y, m et n sont tels que définis précédemment.

Un tel motif peut être obtenu :

- soit par une réaction de condensation entre un silicone à extrémités α, ω-acides carboxyliques et une ou plusieurs diamines, selon le schéma réactionnel suivant :

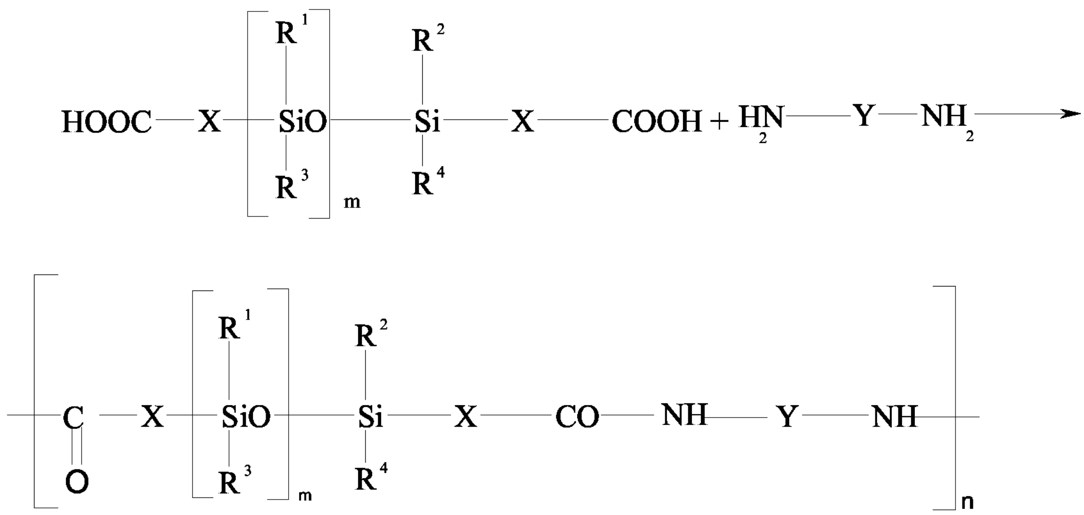

soit par réaction de deux molécules d'acide carboxylique alpha -insaturé avec une diamine selon le schéma réactionnel suivant : $CH_2=CH-X^1-COOH+H_2N-Y-NH_2$ ->$CH_2=CH-X^1-CO-NH-Y-NH-CO-X^1-CH=CH_2$ suivie de l'addition d'un siloxane sur les insaturations éthyléniques, selon le schéma suivant : $CH_2=CH-X^1-CO-NH-Y-NH-CO-X^1-CH=CH_2$ +

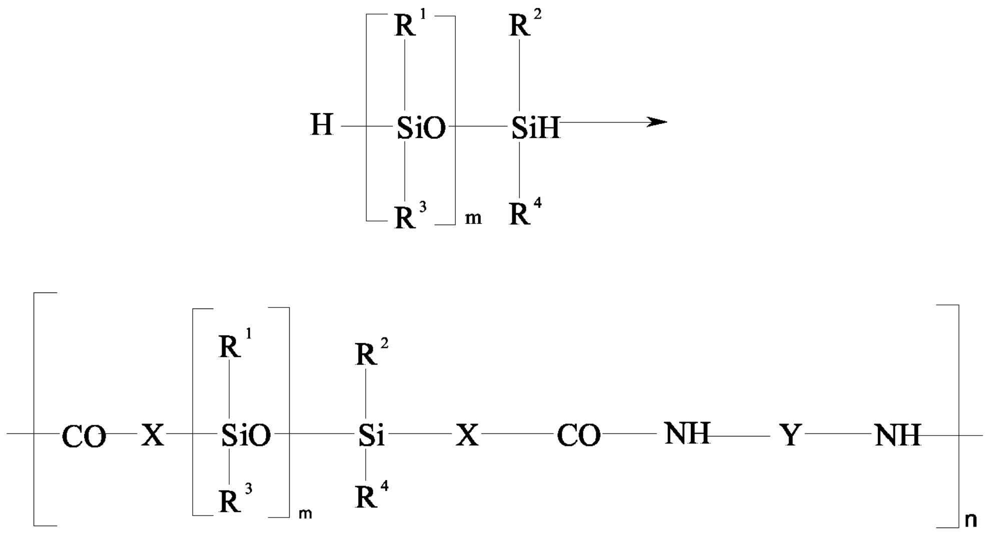

dans lesquels $X^1$-$(CH_2)_2$- correspond au X défini ci-dessus et Y, $R^1$, $R^2$, $R^3$, $R^4$ et m sont tels que définis précédemment ;

- soit par réaction d'un silicone à extrémités α, ω-NH2 et d'un diacide de formule HOOC-Y-COOH selon le schéma réactionnel suivant :

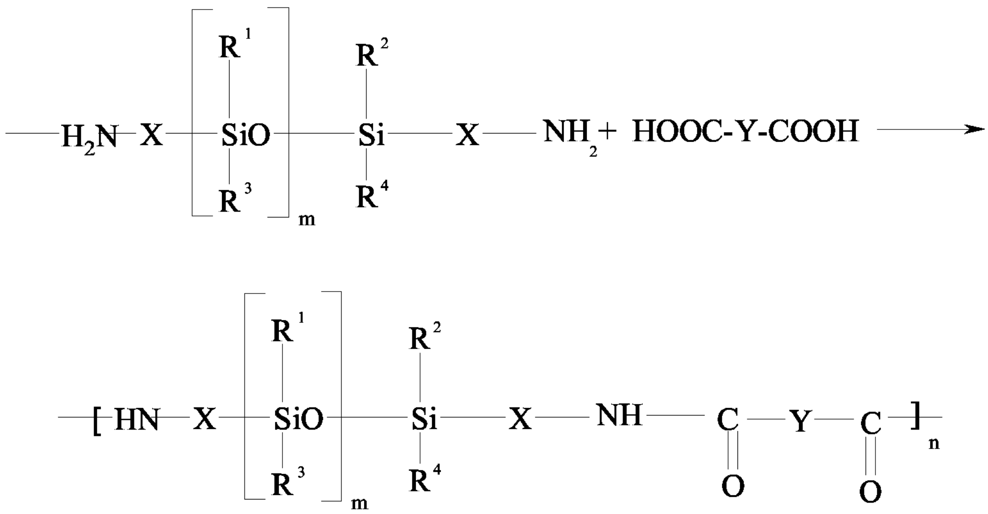

Dans les polyamides selon les troisième et quatrième formules précédemment exposées :

- m est notamment dans la gamme de 1 à 700, voire de 15 à 500 et mieux encore de 15 à 45, et
- n est en particulier dans la gamme de 1 à 500, notamment de 1 à 100 et mieux encore de 4 à 25,
- X est notamment une chaîne alkylène linéaire ou ramifiée ayant de 1 à 30 atomes de carbone, en particulier 3 à 10 atomes de carbone, et
- Y est notamment une chaîne alkylène linéaire ou ramifiée ou pouvant comporter des cycles et/ou des insaturations ayant de 1 à 40 atomes de carbone, en particulier de 1 à 20 atomes de carbone, et mieux encore de 2 à 6 atomes de carbone, en particulier de 6 atomes de carbone.

Dans les troisième et quatrième formules exposées précédemment, le groupe alkylène représentant X ou Y peut éventuellement contenir dans sa partie alkylène au moins l'un des éléments suivants :

1) 1 à 5 groupes amides, urée ou carbamate,

2) un groupe cycloalkyle en $C_5$ ou $C_6$, et
3) un groupe phénylène éventuellement substitué par 1 à 3 groupes alkyles identiques ou différents en $C_1$ à $C_3$.

Dans les troisième et quatrième formules exposées précédemment, les groupes alkylènes peuvent aussi être substitués par au moins un élément choisi dans le groupe constitué de:

- un groupe hydroxy,
- un groupe cycloalkyle en $C_3$ à $C_8$,
- un à trois groupes alkyles en $C_1$ à $C_{40}$,
- un groupe phényle éventuellement substitué par un à trois groupes alkyles en $C_1$ à $C_3$,
- un groupe hydroxyalkyle en $C_1$ à $C_3$, et
- un groupe aminoalkyle en $C_1$ à $C_6$. Dans les troisième et quatrième formules exposées précédemment, Y peut aussi représenter :

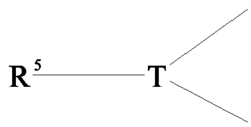

où $R^5$ représente une chaîne polyorganosiloxane, et T représente un groupe de formule :

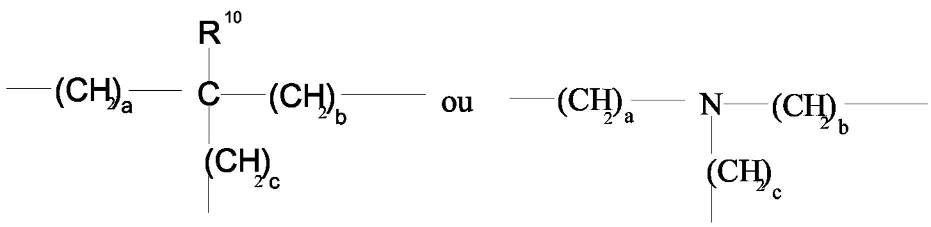

dans lesquelles a, b et c sont, indépendamment, des nombres entiers allant de 1 à 10, et $R^{10}$ est un atome d'hydrogène ou un groupe tel que ceux définis pour $R^1$, $R^2$, $R^3$, $R^4$.

Dans les troisième et quatrième formules exposées précédemment, $R^1$, $R^2$, $R^3$, $R^4$ représentent notamment, indépendamment, un groupe alkyle en $C_1$ à $C_{40}$, linéaire ou ramifié, en particulier un groupe $CH_3$, $C_2H_5$, n-$C_3H_7$ ou isopropyle, une chaîne polyorganosiloxane ou un groupe phényle éventuellement substitué par un à trois groupes méthyle ou éthyle.

Comme on l'a vu précédemment, le polymère peut également comprendre des motifs selon la troisième ou quatrième formule exposées précédemment identiques ou différents.

Ainsi, le polymère peut être un polyamide siliconé contenant plusieurs motifs selon la troisième ou quatrième formule exposées précédemment de longueurs différentes, soit un polyamide répondant à la cinquième formule suivante :

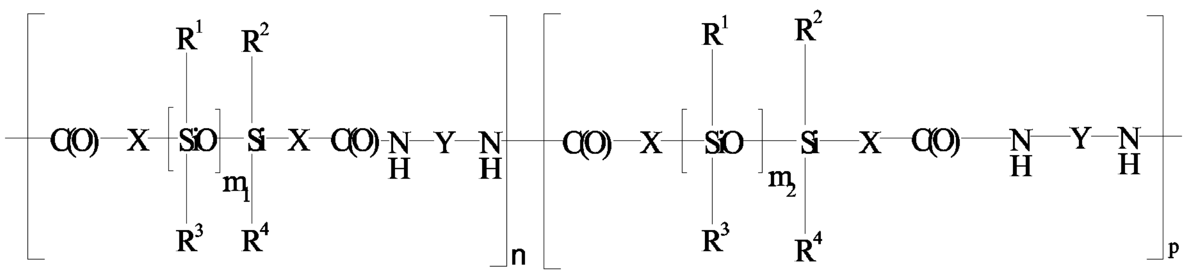

dans laquelle X, Y, n, $R^1$ à $R^4$ ont les significations données précédemment, $m_1$ et $m_2$ qui sont différents, sont choisis dans la gamme allant de 1 à 1000, et p est un nombre entier allant de 2 à 300.

Dans cette formule, les motifs peuvent être structurés pour former soit un copolymère bloc, soit un copolymère

aléatoire, soit un copolymère alterné. Dans ce copolymère, les motifs peuvent être non seulement de longueurs différentes mais aussi de structures chimiques différentes, par exemple ayant des Y différents. Dans ce cas, le copolymère peut répondre à la sixième formule :

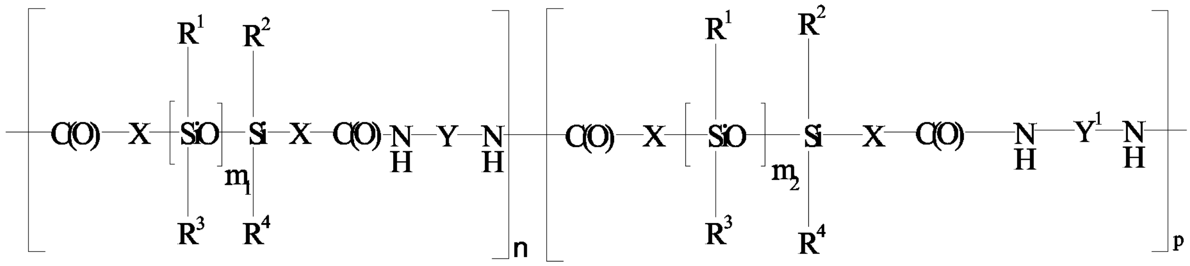

dans laquelle $R^1$ à $R^4$, X, Y, $m_1$, $m_2$, n et p ont les significations données ci-dessus et $Y^1$ est différent de Y, mais est choisi parmi les groupes définis pour Y. Comme précédemment, les différents motifs peuvent être structurés pour former soit un copolymère bloc, soit un copolymère aléatoire, soit un copolymère alterné.

Selon un mode de réalisation de l'invention, le polyamide siliconé gélifiant peut être aussi constitué par un copolymère greffé. Ainsi, le polyamide à unités silicone peut être greffé et éventuellement réticulé par des chaînes silicones à groupes amides. De tels polymères peuvent être synthétisés avec des amines trifonctionnelles.

Dans ce cas, le copolymère peut comprendre au moins un motif selon la septième formule suivante :

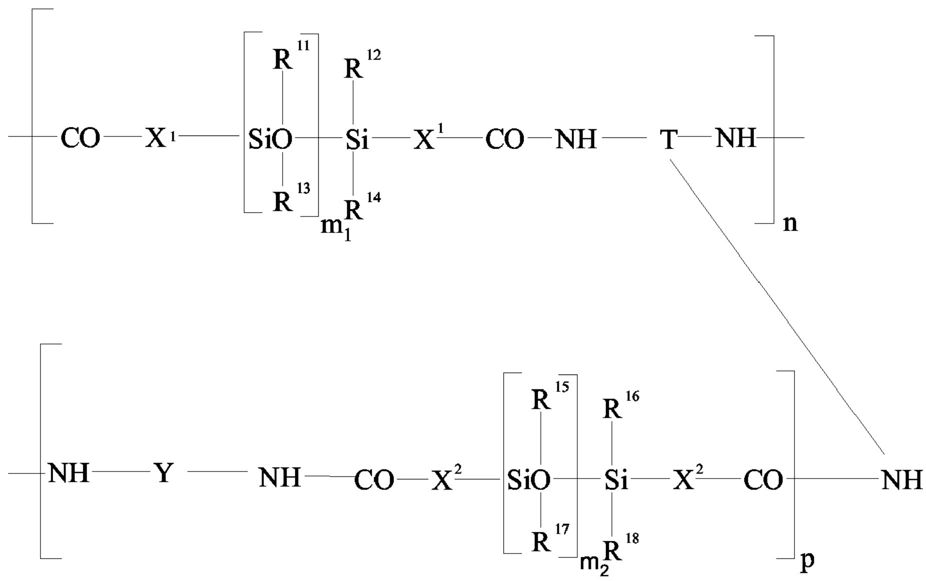

dans laquelle $X^1$ et $X^2$ qui sont identiques, ou différents, ont la signification donnée pour X dans la première formule précédente, n est tel que défini dans la première formule précédente, Y et T sont tels que définis dans la première formule précédente, $R^{11}$ à $R^{18}$ sont des groupes choisis dans le même groupe que les $R^1$ à $R^4$, $m_1$ et $m_2$ sont des nombres situés dans la gamme allant de 1 à 1 000, et p est un nombre entier allant de 2 à 500.

Dans la septième formule précédemment exposée, en particulier :

- p est dans la gamme de 1 à 25, mieux encore de 1 à 7,
- $R^{11}$ à $R^{18}$ sont des groupes méthyle,
- T répond à l'une des formules suivantes :

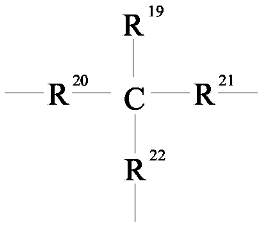 ; 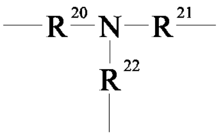 ; 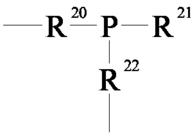

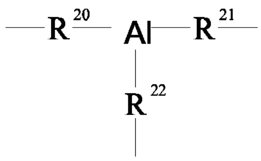

dans lesquelles $R^{19}$ est un atome d'hydrogène ou un groupe choisi parmi les groupes définis pour $R^1$ à $R^4$, et $R^{20}$, $R^{21}$ et $R^{22}$ sont, indépendamment, des groupes alkylène, linéaires ou ramifiés,
T répond en particulier de préférence à la formule :

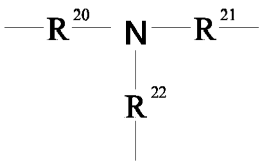

avec notamment $R^{20}$, $R^{21}$ et $R^{22}$ représentant $-CH_2-CH_2-$,

- $m_1$ et $m_2$ sont dans la gamme de 15 à 500, voire de 15 à 45,
- $-X^1$ et $X^2$ représentent $-(CH_2)_{10}-$, et
- Y représente $-CH_2-$.

Ces polyamides à motif silicone greffé selon la septième formule exposée précédemment peuvent être copolymérisés avec des polyamides-silicones selon la seconde formule pour former des copolymères blocs, des copolymères alternés ou des copolymères aléatoires. Le pourcentage en poids de motifs siliconés greffés selon la septième formule dans le copolymère peut aller de 0,5 à 30 % en poids.

Selon un mode de réalisation, les unités siloxanes peuvent être dans la chaîne principale ou squelette du polymère, mais elles peuvent également être présentes dans des chaînes greffées ou pendantes. Dans la chaîne principale, les unités siloxanes peuvent être sous forme de segments comme décrits ci-dessus. Dans les chaînes pendantes ou greffées, les unités siloxanes peuvent apparaître individuellement ou en segments.

Selon un mode de réalisation de l'invention, les polyamides à base de siloxanes peuvent notamment être :

- les polyamides selon la troisième formule précédemment exposée où m est de 15 à 50 ;
- les mélanges de deux ou plusieurs polyamides dans lesquels au moins un polyamide a une valeur de m dans la gamme de 15 à 50 et au moins un polyamide a une valeur de m dans la gamme de 30 à 50 ; des polymères selon la cinquième formule décrite précédemment avec $m_1$ choisi dans la gamme de 15 à 50 et $m_2$ choisi dans la gamme de 30 à 500 avec la partie correspondant à $m_1$ représentant 1 à 99 % en poids du poids total du polyamide et la partie correspondant à $m_2$ représentant 1 à 99 % en poids du poids total du polyamide ;
- des mélanges de polyamide selon la troisième formule décrite précédemment combinant :

    1) 80 à 99 % en poids d'un polyamide où n est égal à 2 à 10, en particulier 3 à 6, et
    2) 1 à 20 % d'un polyamide où n est dans la gamme de 5 à 500, en particulier de 6 à 100 ;

- des polyamides répondant à la sixième formule précédemment exposée où au moins l'un des Y et $Y^1$ contient au moins un substituant hydroxyle.
- des polyamides selon la troisième formule synthétisés avec au moins une partie d'un diacide activé (chlorure,

dianhydride ou diester de diacide) au lieu du diacide ;

- des polyamides selon la troisième formule où X représente - $(CH_2)_3$- ou -$(CH_2)_{10}$; et
- des polyamides selon la troisième formule où les polyamides sont terminés par une chaîne monofonctionnelle choisie dans le groupe constitué des amines monofonctionnelles, des acides monofonctionnels, des alcools monofonctionnels, incluant les acides gras, les alcools gras et les amines grasses, tels que par exemple l'octylamine, l'octanol, l'acide stéarique et l'alcool stéarylique.

Selon un mode de réalisation de l'invention, les extrémités des chaînes du polymère peuvent être terminées par :

- un groupe ester d'alkyle en $C_1$ à $C_{50}$ en introduisant en cours de synthèse un monoalcool en $C_1$ en $C_{50}$,
- un groupe amide d'alkyle en $C_1$ à $C_{50}$ en prenant comme stoppeur un monoacide si la silicone est alpha , omega -diaminée, ou une monoamine si la silicone est alpha, omega -diacide carboxylique.

Selon une autre variante de réalisation de l'invention, on peut utiliser un copolymère de polyamide silicone et de polyamide hydrocarboné, soit un copolymère comportant des motifs selon la troisième ou la quatrième formule et des motifs polyamide hydrocarboné. Dans ce cas, les motifs polyamide-silicone peuvent être disposés aux extrémités du polyamide hydrocarboné.

Des agents gélifiants à base de polyamide contenant des silicones peuvent être produits par amidation silylique de polyamides à base de dimère d'acide gras. Cette approche implique la réaction de sites acides libres existant sur un polyamide comme sites terminaux, avec des oligosiloxanes-monoamines et/ou des oligosiloxanes-diamines (réaction d'amidation), ou alternativement avec des oligosiloxanes alcools ou des oligosiloxanes diols (réaction d'estérification). La réaction d'estérification nécessite la présence de catalyseurs acides, comme il est connu dans la technique. Il est souhaitable que le polyamide ayant des sites acides libres, utilisés pour la réaction d'amidation ou d'estérification, ait un nombre relativement élevé de terminaisons acides (par exemple des polyamides ayant des indices d'acide élevés, par exemple de 15 à 20).

Pour l'amidation des sites acides libres des polyamides hydrocarbonés, des siloxanes diamines avec 1 à 300, plus particulièrement 2 à 50, et mieux encore 2, 6, 9, 5, 12, 13,5, 23 ou 31 groupes siloxanes, peuvent être utilisés pour la réaction avec des polyamides hydrocarbonés à base de dimères d'acide gras. On préfère des siloxanes diamines ayant 13,5 groupes siloxanes et les meilleurs résultats sont obtenus avec la siloxane-diamine ayant 13,5 groupes siloxane et des polyamides contenant des indices élevés de groupes terminaux acides carboxyliques.

Les réactions peuvent être effectuées dans le xylène pour extraire l'eau produite de la solution par distillation azéotropique, ou à des températures plus élevées (autour de 180 à 200 °C) sans solvant. Typiquement, l'efficacité de l'amidation et les taux de réaction diminuent lorsque le siloxane diamine est plus long, c'est-à-dire lorsque le nombre de groupes siloxanes est plus élevé. Des sites amines libres peuvent être bloqués après la réaction d'amidation initiale des diaminosiloxanes en les faisant réagir avec soit un siloxane acide, soit un acide organique tel que l'acide benzoïque.

Pour l'estérification des sites acides libres sur les polyamides, ceci peut être réalisé dans le xylène bouillant avec environ 1 % en poids, par rapport au poids total des réactifs, d'acide paratoluènesulfonique comme catalyseur.

Ces réactions effectuées sur les groupes acides carboxyliques terminaux du polyamide conduisent à l'incorporation de motifs silicone seulement aux extrémités de la chaîne de polymère.

A titre d'exemple de polymère gélifiant de type polyamide siliconé convenant à la mise en oeuvre de l'invention, on peut mentionner le copolymère bloc polyamide/polydiméthylsiloxane par exemple vendu sous la référence DC2-8178 GELLANT par la société DOW CORNING. (Nom INCI Nylon-611/dimethicone copolymer (and) PPG-3 myristyl ether).

<u>*Mono ou polyalkylesters de polysaccharide*</u>

Parmi les mono ou polyalkylesters de saccharide ou de polysaccharide convenant à la mise en oeuvre de l'invention, on peut mentionner les alkyles ou polyalkylesters de dextrine ou d'inuline.

Il peut s'agir notamment d'un mono-ou poly-ester de dextrine et d'au moins un acide gras et notamment répondant à la formule suivante :

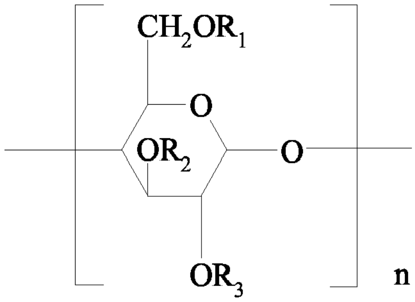

dans laquelle :

- n est un entier allant de 3 à 200, notamment allant de 20 à 150, et en particulier allant de 25 à 50,
- les radicaux $R_1$, $R_2$ et $R_3$, identiques ou différents, sont choisis parmi l'hydrogène ou un groupement acyle (R-CO-) dans lequel le radical R est un groupement hydrocarboné, linéaire ou ramifié, saturé ou insaturé, possédant de 7 à 29, en particulier de 7 à 21, notamment de 11 à 19, plus particulièrement de 13 à 17, voire 15, atomes de carbone, sous réserve qu'au moins un desdits radicaux $R_1$, $R_2$ ou $R_3$ est différent de l'hydrogène.

En particulier, $R_1$, $R_2$ et $R_3$ peuvent représenter l'hydrogène ou un groupement acyle (R-CO-) dans lequel R est un radical hydrocarboné tel que défini précédemment, sous réserve qu'au moins deux desdits radicaux $R_1$, $R_2$ ou $R_3$ sont identiques et différents de l'hydrogène.
L'ensemble des radicaux $R_1$, $R_2$ et $R_3$ peut figurer un groupement acyle (R-CO) identique ou différent, et notamment identique.
En particulier, n précédemment exposé varie avantageusement de 25 à 50, notamment est égal à 38 dans la formule générale de l'ester de saccharide utilisable dans la présente invention.
Notamment lorsque les radicaux $R_1$, $R_2$ et/ou $R_3$, identiques ou différents figurent un groupement acyle (R-CO), ceux-ci peuvent être choisis parmi les radicaux caprylique, caprique, laurique, myristique, palmitique, stéarique, arachique, béhénique, isobutyrique, isovalérique, éthyl-2 butyrique, éthylméthylacétique, isoheptanoïque, éthyl-2 hexanoïque, isononanoïque, isodécanoïque, isotridécanoïque, isomyristique, isopalmitique, isostéarique, isoaracique, isohexanoïque, décénoïque, dodécenoïque, tetradécénoïque, myristoléïque, hexadécénoïque, palmitoléïque, oléïque, élaidique, asclépinique, gondoléïque, eicosènoïque, sorbique, linoléïque, linolénique, punicique, stéaridonique, arachidonique, stéarolique, et leurs mélanges.
De préférence, on utilise à titre d'ester de dextrine et d'acide(s) gras au moins un palmitate de dextrine. Celui-ci peut être utilisé seul ou en mélange avec d'autres esters.
Avantageusement, l'ester de dextrine et d'acide gras a un degré de substitution inférieur ou égal à 2,5 sur la base d'une unité glucose, notamment variant de 1,5 à 2,5, de préférence de 2 à 2,5. Le poids moléculaire moyen en poids de l'ester de dextrine peut être en particulier de 10 000 à 150 000, notamment de 12 000 à 100 000 et voire de 15 000 à 80 000
Des esters de dextrine, en particulier des palmitates de dextrine, sont disponibles commercialement sous la dénomination RHEOPEARL TL ou RHEOPEARL KL de la société CHIBA FLOUR.

*Copolymères di-bloc, tri-bloc, multi-bloc, radial-bloc ou en étoile*

Les polymères bloc convenant à la mise en oeuvre de l'invention sont notamment ceux décrits dans les brevets US 5,756,082 et EP 0 497 144, ainsi que dans la demande WO 98/422,98, incorporés dans la présente demande par référence.
Egalement, les polymères bloc utilisables dans la présente invention peuvent être choisis parmi :

- les copolymères bloc (di- ou tri-bloc), tels que les polystyrènes silicones, ou les polyéthylènes silicones, décrites dans les brevets US 6,225,390, US 6,160,054, US 6,174,968 et US 6,225,390,
- les copolymères bloc ou greffés comprenant une séquence siliconée et une autre séquence ou greffon de type polyvinyle ou polyméthacrylique, tels que ceux décrits dans les brevets US 5,468,477 et US 5,725,882,
- les polymères ou copolymères résultant de la polymérisation ou copolymérisation d'un monomère éthylénique, comprenant une ou plusieurs liaisons éthyléniques, optionnellement conjuguées (ou diènes),
- les polymères ou copolymères résultant de la polymérisation ou copolymérisation d'un monomère éthylénique,

notamment un copolymère de type vinyle, acrylique ou méthacrylique, qui peut être un copolymère bloc, tel qu'un copolymère di-bloc ou tri-bloc, ou même multi-bloc ou un copolymère radial ou étoilé.

L'agent gélifiant de type éthylénique peut comprendre, par exemple, un bloc styrène, un bloc alkyle-styrène, un bloc éthylène/butylène, un bloc éthylène/propylène, un bloc butadiène, un bloc isoprène, un bloc acrylate, un bloc méthacrylate, ou une combinaison de ces blocs.

Selon un mode de réalisation, les copolymères di-bloc, tri-bloc, multi-bloc et/ou radial ou étoilé peuvent comporter au moins deux segments incompatibles du point de vue thermodynamique.

Un copolymère di-bloc est habituellement défini comme étant de type A-B ou comme une séquence dans laquelle un segment dur (A) est suivi par un segment souple (B).

Un copolymère tri-bloc est habituellement défini comme étant de type A-B-A ou comme un rapport d'un segment dur, d'un segment souple et d'un segment dur.

Un copolymère multi-bloc ou radial ou étoilé peut comporter n'importe quel type de combinaison de segments durs et de segments souples, sous réserve que les caractéristiques des segments durs et des segments souples soient conservées.

A titre d'exemple de segments durs de copolymère bloc, il peut être fait mention du styrène, et à titre d'exemple de segments souples de copolymère bloc, il peut être fait mention de l'éthylène, du propylène, du butylène, et d'une combinaison de ceux-ci.

Les copolymères tri-bloc, et notamment ceux de type polystyrène/polyisoprène ou polystyrène/polybutadiène, convenant à la mise en oeuvre de l'invention peuvent être ceux commercialisés sous la référence LUVITOL HSB par la société BASF. Il peut également être fait mention des copolymères tri-bloc de type polystyrène/copoly(éthylènepropylène) ou polystyrène/ copoly(éthylène -butylène), tels que ceux commercialisés sous la référence KRATON par la société SHELL CHEMICAL CO, ou sous la référence GELLED PERMETHYL 99 A par la société PENRECO.

A titre d'exemple, encore, de copolymères bloc susceptibles de convenir à la mise en oeuvre de la présente invention, il peut également être fait mention des copolymères blocs commercialisés sous la référence VERSAGEL par la société PENRECO, ceux commercialisés sous la référence KRATON par la société SHELL et ceux commercialisés sous la référence GEL BASE par la société BROOKS INDUSTRIES.

Parmi les polymères gélifiants de phase huileuse convenant à la mise en oeuvre de l'invention, on peut notamment citer le copolymère d'éthylènediamine/stéaryle dimerdilinoléate, le polymère semi-cristallin de type Poly-$C_{10-30}$ alkylacrylate, et leurs mélanges.

Selon une variante de réalisation de la présente invention, les particules huileuses calibrées structurées peuvent contenir, notamment, en tant qu'huile, du lauroylesarcosinate d'isopropyle, et en tant que polymère gélifiant, un copolymère de dimerdilinoleate d'éthylènediamine/stéaryle.

Selon une autre variante de réalisation, les particules huileuses calibrées structurées peuvent comprendre en tant qu'huile, du squalane et en tant que polymère gélifiant, un polymère semi-cristallin de type poly-$C_{10-30}$ alkylacrylate.

Selon encore un autre mode de réalisation, les particules huileuses calibrées structurées peuvent comprendre en tant que phase huileuse un mélange de laurylsarcosinate d'isopropyle et d'isononanoate d'isononyle et en tant que polymère gélifiant le copolymère dimerdilinoleate d'éthylènediamine/stéaryle.

Selon encore un autre mode de réalisation, les particules huileuses calibrées structurées conformes à la présente invention peuvent comprendre en tant que phase huileuse un mélange d'isononanoate d'isononyle et d'octyldodécanol et en tant que polymère gélifiant, le copolymère dimerdilinoleate d'éthylènediamine/stéaryle.

En outre, les particules peuvent comprendre un autre gélifiant de type dérivé amide d'acide alcoyle glutamique, par exemple le dibutylamide d'acide laurylglutamique, commercialisé par la société AJINOMOTO sous le nom "GELLING AGENT GP-1".

## FILTRES SOLAIRES

Les particules conformes à l'invention comportent au moins un filtre solaire hydrophobe qui peut être choisi parmi un agent photoprotecteur organique hydrophobe, un agent photoprotecteur inorganique actif dans l'UVA et/ou l'UVB (absorbeurs), et leurs mélanges.

Les filtres organiques hydrophobes sont notamment choisis parmi les anthranilates ; les dérivés cinnamiques ; les dérivés de dibenzoylméthane; les dérivés salicyliques, les dérivés du camphre ; les dérivés de triazine tels que ceux décrits dans les demandes de brevet US 4 367 390, EP 863 145, EP 517 104, EP 570 838, EP 796 851, EP 775 698, EP 878 469, EP 933 376, EP 507 691, EP 507 692, EP 790 243, EP 944 624 ; les dérivés de la benzophénone ; les dérivés de β,β-diphénylacrylate; les dérivés de benzotriazole ; les dérivés de benzalmalonate ; les dérivés de benzimidazole ; les imidazolines ; les dérivés bis-benzoazolyle tels que décrits dans les brevets EP 669 323 et US 2,463,264; les dérivés de l'acide p-aminobenzoïque (PABA) ; les dérivés de méthylène bis-(hydroxyphényl benzotriazole) tels que décrits dans les demandes US 5,237,071, US 5,166,355, GB 2 303 549, DE 197 26 184 et EP 893 119 ; les

polymères filtres et silicones filtres tels que ceux décrits notamment dans la demande WO-93/04665 ; les dimères dérivés d'$\alpha$-alkylstyrène tels que ceux décrits dans la demande de brevet DE 19 855 649 ; les 4,4-diarylbutadiènes tels que décrits dans les demandes EP 0 967 200, DE 19 746 654, DE 19 755 649, EP-A-1 008 586, EP 1 133 980 et EP 133 981 et leurs mélanges.

Comme exemples de filtres organiques hydrophobes, on peut citer ceux désignés ci-dessous sous leur nom INCI :

<u>Dérivés de l'acide para-aminobenzoïque :</u>

- Ethyl PABA,
- Ethyl Dihydroxypropyl PABA,
- Ethylhexyl Diméthyl PABA vendu notamment sous le nom "ESCALOL 507" par ISP,

<u>Dérivés salicyliques :</u>

- Homosalate vendu sous le nom "Eusolex HMS" par Rona/EM Industries,
- Ethylhexyl Salicylate vendu sous le nom "NEO HELIOPAN OS" par HAARMANN et REIMER,

<u>Dérivés du dibenzoylméthane</u> :

- Butyl Methoxydibenzoylmethane vendu notamment sous le nom commercial "PARSOL 1789" par HOFFMANN LA ROCHE,
- Isopropyl Dibenzoylmethane,

<u>Dérivés cinnamiques :</u>

- Ethylhexyl Methoxycinnamate vendu notamment sous le nom commercial "PARSOL MCX" par HOFFMANN LA ROCHE,
- Isopropyl Methoxy cinnamate,
- Isoamyl Methoxy cinnamate vendu sous le nom commercial "NEO HELIOPAN E 1000" par HAARMANN et REIMER,
- Diisopropyl Methylcinnamate,

<u>Dérivés de $\beta,\beta'$-diphénylacrylate</u> :

- Octocrylene, vendu notamment sous le nom commercial "UVINUL N539" par BASF,
- Etocrylene, vendu notamment sous le nom commercial "UVINUL N35" par BASF,

<u>Dérivés de la benzophénone :</u>

- Benzophenone-1 vendu sous le nom commercial "UVINUL 400" par BASF,
- Benzophenone-2 vendu sous le nom commercial "UVINUL D50" par BASF
- Benzophenone-3 ou Oxybenzone, vendu sous le nom commercial "UVINUL M40" par BASF,
- Benzophenone-6 vendu sous le nom commercial "Helisorb 11" par Norquay
- Benzophenone-8 vendu sous le nom commercial "Spectra-Sorb UV-24" par American Cyanamid
- Benzophenone-10,
- Benzophenone-11,
- Benzophenone-12,
- Diethylamino Hydroxybenzoyl Hexyl Benzoate vendu sous le nom commercial "UVINUL A PLUS" par BASF,

<u>Dérivés du benzylidène camphre :</u>

- 3-Benzylidène camphre fabriqué sous le nom "MEXORYL SD" par CHIMEX,
- Méthylbenzylidène camphre vendu sous le nom "EUSOLEX 6300" par MERCK,
- Polyacrylamidométhyle Benzylidène Camphre fabriqué sous le nom "MEXORYL SW" par CHIMEX,

<u>Dérivés de la triazine :</u>

- Bis-Ethylhexyloxyphenol Methoxyphenyl Triazine vendu sous le nom commercial "TINOSORB S" par CIBA GEIGY,
- Ethylhexyl triazone vendu notamment sous le nom commercial "UVINUL T150" par BASF,

- Diethylhexyl Butamido Triazone vendu sous le nom commercial "UVASORB HEB" par SIGMA 3V,
- 2,4,6-tris(4'-amino benzalmalonate de dinéopentyle)-s-triazine
- la 2,4,6-tris-(4'-amino benzalmalonate de diisobutyle)-s- triazine.

Dérivés du phenyl benzotriazole:

- Drometrizole Trisiloxane vendu sous le nom "Silatrizole" par RHODIA CHIMIE Methylène bis-Benzotriazolyl Tetramethylbutylphénol, vendu sous forme solide sous le nom commercial "MIXXIM BB/100" par FAIRMOUNT CHEMICAL ou sous forme micronisé en dispersion aqueuse sous le nom commercial "TINOSORB M" par CIBA SPECIALTY CHEMICALS,

Dérivés anthraniliques :

- Menthyl anthranilate vendu sous le nom commercial commercial "NEO HELIOPAN MA" par HAARMANN et REIMER,

Dérivés d'imidazolines :

- Ethylhexyl Dimethoxybenzylidene Dioxoimidazoline Propionate,

Dérivés du benzalmalonate :

- Polyorganosiloxanes à fonction benzalmalonate tels que le Polysilicone-15 vendu sous la dénomination commerciale "PARSOL SLX" par HOFFMANN LA ROCHE

Dérivés de 4,4-diarylbutadiène :

- 1,1-dicarboxy (2,2'-diméthyl-propyl)-4,4-diphénylbutadiène,

Dérivés de benzoxazole :

- 2,4-bis-[5-1(diméthylpropyl)benzoxazol-2-yl-(4-phenyl)-imino]-6-(2-ethylhexyl)-imino-1,3,5-triazine vendu sous le nom d'Uvasorb K2A par Sigma 3V
- et leurs mélanges.

Les filtres UV organiques hydrophobes préférentiels sont choisis parmi :

- Ethylhexyl Salicylate,
- Homosalate,
- Ethylhexyl Methoxycinnamate,
- Butyl Methoxydibenzoylmethane,
- Octocrylene,
- Benzophenone-3,
- 2-(4-diethylamino-2-hydroxybenzoyl)-benzoate de n-hexyle,
- 4-Methylbenzylidene camphor,
- Bis-Ethylhexyloxyphenol Methoxyphenyl Triazine,
- Ethylhexyl triazone,
- Diethylhexyl Butamido Triazone,
- Methylène bis-Benzotriazolyl Tetramethylbutylphénol,
- Drometrizole Trisiloxane,
- Polysilicone-15,
- 1,1-dicarboxy (2,2'-diméthyl-propyl)-4,4-diphénylbutadiène,
- 2,4-bis-[5-1(diméthylpropyl)benzoxazol-2-yl-(4-phenyl)-imino]-6-(2-ethylhexyl)-imino-1,3,5-triazine,
- et leurs mélanges.

Les particules conformes à l'invention peuvent comprendre des agents protecteurs inorganiques. Toutefois, ils sont de préférence présents en de faibles quantités comme cela est détaillé ci-après.

Les agents photoprotecteurs inorganiques sont choisis parmi des pigments et plus préférentiellement encore des nanopigments (taille moyenne des particules primaires : généralement entre 5 nm et 100 nm, de préférence entre

10 nm et 50 nm) d'oxydes métalliques traités ou non comme par exemple des nanopigments d'oxyde de titane (amorphe ou cristallisé sous forme rutile et/ou anatase), de fer, de zinc, de zirconium ou de cérium.

Les nanopigments traités sont des pigments qui ont subi un ou plusieurs traitements de surface de nature chimique, électronique, mécanochimique et/ou mécanique avec des composés tels que décrits par exemple dans Cosmetics & Toiletries, Février 1990, Vol. 105, p 53-64, tels que des aminoacides, de la cire d'abeille, des acides gras, des alcools gras, des tensio-actifs anioniques, des lécithines, des sels de sodium, potassium, zinc, fer, ou aluminium d'acides gras, des alcoxydes métalliques (de titane ou d'aluminium), du polyéthylène, des silicones, des protéines (collagène, élastine) des alcanolamines, des oxydes de silicium, des oxydes métalliques, de l'hexamétaphosphate de sodium, de l'alumine ou de la glycérine.

Les nanopigments traités peuvent être plus particulièrement des oxydes de titane traités par :

- la silice et l'alumine tels que les produits "Microtitanium Dioxide MT 500 SA" et "Microtitanium Dioxide MT 100 SA" de la société TAYCA, et les produits "Tioveil Fin", "Tioveil OP", "Tioveil MOTG" et "Tioveil IPM" de la société TIOXIDE,
- l'alumine et le stéarate d'aluminium tels que le produit "Microtitanium Dioxide MT 100 T" de la société TAYCA,
- l'alumine et le laurate d'aluminium tels que le produit "Microtitanium Dioxide MT 100 S" de la société TAYCA,
- des oxydes de fer et le stéarate de fer tels que le produit "Microtitanium Dioxide MT 100 F" de la société TAYCA,
- la silice, l'alumine et la silicone tels que les produits "Microtitanium Dioxide MT 100 SAS", "Microtitanium Dioxide MT 600 SAS" et "Microtitanium Dioxide MT 500 SAS" de la société TAYCA,
- l'hexamétaphosphate de sodium tels que le produit "Microtitanium Dioxide MT 150 W" de la société TAYCA,
- l'octyltriméthoxysilane tels que le produit "T-805" de la société DEGUSSA,
- l'alumine et l'acide stéarique tels que le produit "UVT-M160" de la société KEMIRA,
- l'alumine et la glycérine tels que le produit "UVT-M212" de la société KEMIRA,
- l'alumine et la silicone tels que le produit "UVT-M262" de la société KEMIRA.

D'autres nanopigments d'oxyde de titane traités avec une silicone sont de préférence le $TiO_2$ traité par l'octyl triméthyl silane et dont la taille moyenne des particules élémentaires est comprise entre 25 et 40 nm tel que celui vendu sous la dénomination commerciale "T 805" par la société DEGUSSA SILICES, le $TiO_2$ traité par un polydiméthylsiloxane et dont la taille moyenne des particules élémentaires est de 21 nm tel que celui vendu sous la dénomination commerciale "70250 Cardre UF TiO2SI3" par la société CARDRE, le $TiO_2$ anatase/rutile traité par un polydiméthylhydrogénosiloxane et dont la taille moyenne des particules élémentaires est de 25 nm tel que celui vendu sous la dénomination commerciale "MICRO TITANIUM DIOXYDE USP GRADE HYDROPHOBIC" par la société COLOR TECHNIQUES.

Les nanopigments d'oxyde de titane non enrobés sont par exemple vendus par la société TAYCA sous les dénominations commerciales "MICROTITANIUM DIOXIDE MT 500 B" ou "MICROTITANIUM DIOXIDE MT600 B", par la société DEGUSSA sous la dénomination "P 25", par la société WACKHER sous la dénomination "Oxyde de titane transparent PW", par la société MIYOSHI KASEI sous la dénomination "UFTR", par la société TOMEN sous la dénomination "ITS" et par la société TIOXIDE sous la dénomination "TIOVEIL AQ".

Les nanopigments d'oxyde de zinc non enrobés, sont par exemple :

- ceux commercialisés sous la dénomination "Z-cote" par la société Sunsmart ;
- ceux commercialisés sous la dénomination "Nanox" par la société Elementis ;
- ceux commercialisés sous la dénomination "Nanogard WCD 2025" par la société Nanophase Technologies ;

Les nanopigments d'oxyde de zinc enrobés sont par exemple

- ceux commercialisés sous la dénomination "Oxide zinc CS-5" par la société Toshibi (ZnO enrobé par polymethylhydrogenesiloxane) ;
- ceux commercialisés sous la dénomination "Nanogard Zinc Oxide FN" par la société Nanophase Technologies (en dispersion à 40 % dans le Finsolv TN, benzoate d'alcools en $C_{12}$-$C_{15}$) ;
- ceux commercialisés sous la dénomination "DAITOPERSION ZN-30" et "DAITOPERSION Zn-50" par la société Daito (dispersions dans cyclopolyméthylsiloxane /polydiméthylsiloxane oxyéthyléné, contenant 30 % ou 50 % de nano-oxydes de zinc enrobés par la silice et le polyméthylhydrogènesiloxane) ;
- ceux commercialisés sous la dénomination "NFD Ultrafine ZnO" par la société Daikin (ZnO enrobé par phosphate de perfluoroalkyle et copolymère à base de perfluoroalkyléthyle en dispersion dans du cyclopentasiloxane) ;
- ceux commercialisés sous la dénomination "SPD-Z1" par la société Shin-Etsu (ZnO enrobé par polymère acrylique greffé silicone, dispersé dans cyclodiméthylsiloxane) ;
- ceux commercialisés sous la dénomination "Escalol Z100" par la société ISP (ZnO traité alumine et dispersé dans le mélange methoxycinnamate d'éthylhexyle /copolymère PVP-hexadecene / methicone) ;
- ceux commercialisés sous la dénomination "Fuji ZnO-SMS-10" par la société Fuji Pigment (ZnO enrobé silice et

polymethylsilsesquioxane) ;

- ceux commercialisés sous la dénomination "Nanox Gel TN" par la société Elementis (ZnO dispersé à 55 % dans du benzoate d'alcools en $C_{12}$-$C_{15}$ avec polycondensat d'acide hydroxystéarique).

Les nanopigments d'oxyde de cérium non enrobé est vendu sous la dénomination "COLLOIDAL CERIUM OXIDE" par la société RHONE POULENC.

Les nanopigments d'oxyde de fer non enrobés sont par exemple vendus par la société ARNAUD sous les dénominations "NANOGARD WCD 2002 (FE 45B)", "NANOGARD IRON FE 45 BL AQ", "NANOGARD FE 45R AQ, "NANOGARD WCD 2006 (FE 45R)", ou par la société MITSUBISHI sous la dénomination "TY-220".

Les nanopigments d'oxyde de fer enrobés sont par exemple vendus par la société ARNAUD sous les dénominations "NANOGARD WCD 2008 (FE 45B FN)", "NANOGARD WCD 2009 (FE 45B 556)", "NANOGARD FE 45 BL 345", "NANOGARD FE 45 BL", ou par la société BASF sous la dénomination "OXYDE DE FER TRANSPARENT".

On peut également citer les mélanges d'oxydes métalliques, notamment de dioxyde de titane et de dioxyde de cérium, dont le mélange équipondéral de dioxyde de titane et de dioxyde de cérium enrobés de silice, vendu par la société IKEDA sous la dénomination "SUNVEIL A", ainsi que le mélange de dioxyde de titane et de dioxyde de zinc enrobé d'alumine, de silice et de silicone tel que le produit "M 261" vendu par la société KEMIRA ou enrobé d'alumine, de silice et de glycérine tel que le produit "M 211" vendu par la société KEMIRA.

Les nanopigments peuvent être introduits dans les particules selon l'invention tels quels ou sous forme de pâte pigmentaire, c'est-à-dire en mélange avec un dispersant, comme décrit par exemple dans le document GB-A-2206339.

La teneur en filtre(s) solaire(s) organique(s) hydrophobe(s) présent(s) dans les particules conformes à l'invention peut varier de 0,05 à 80 % en poids, de 0,1 à 60 % en poids et mieux de 0,5 à 50 % en poids, par rapport au poids total de la particule.

La teneur en filtres solaires inorganiques présent(s) dans les particules peut varier entre 0.05 et 20 % en poids et préférentiellement entre 0.1 et 10 % en poids, par rapport au poids total de la particule.

A titre optionnel, les particules peuvent contenir en outre au moins une substance active lipophile additionnelle, présentant une activité biologique apparentée ou non à la photoprotection.

L'utilisation de particules calibrées conformes à l'invention permet d'améliorer significativement le facteur de protection solaire (SPF) comparativement à une émulsion dont la phase huileuse est gélifiée ou non par les mêmes gélifiants huileux.

Le SPF est avantageusement déterminé selon la méthode "in vitro" décrite par B.L. Diffey dans J. Soc. Cosmet. Chem. 40, 127-133 (1989).

## AGENTS TENSIOACTIFS

Les particules selon l'invention peuvent en outre comprendre au moins un tensioactif.

La présence de tensioactif(s) et leur nature chimique sont généralement déterminés par la nature du procédé de préparation desdites particules.

Ainsi, lorsque les particules sont préparées selon le procédé décrit ci-après, impliquant une étape d'émulsification, il est introduit dans le mélange phase huileuse-phase aqueuse, au moins un agent tensioactif choisi parmi les agents tensioactifs non-ioniques, les agents tensioactifs ioniques, et leur mélanges.

Les tensioactifs non-ioniques, ou leurs mélanges, avantageusement utilisés dans le cadre de la présente invention sont des tensioactifs, ou leurs mélanges, ayant une HLB supérieur à 5.

A titre d'exemple de tensioactifs non-ioniques convenant pour la mise en oeuvre de l'invention, on peut citer les :

- mono- ou polyalkylesters ou éthers de glycérol oxyethyléné, ou non, tels que ceux décrits dans le brevet US 6 541 018 ;
- mono- ou polyalkylesters ou éthers de sorbitan oxyéthyléné, ou non, tels que ceux décrits dans US 6 335 022 ;
- mono- ou polyalkylesters ou éthers de polyoxyde d'éthylène, tels que ceux décrits dans US 6 375 960 ;
- mono- ou polyalkylesters ou éthers de sucre oxyethyléné, ou non, tels que ceux décrits dans US 6 689 371 ; et
- leurs mélanges.

Les tensioactifs ioniques utilisables dans le cadre de la présente invention peuvent être de type anionique, de type cationique ou de type amphiphile.

Les tensioactifs anioniques peuvent être, notamment, choisis parmi :

- les alkenyl succinate alkoxylés tels que ceux cités dans le brevet US 6 461 625 ;
- les alkyl éther citrates tels que ceux cités dans le brevet US 6 413 527 ;
- les alkyl ester phosphoriques tels que ceux décrits dans le brevet US 6 274 150 ; et

- leurs mélanges.

Les chaînes alkyles des tensioactifs anioniques convenant à la mise en oeuvre de l'invention sont avantageusement comprises dans l'intervalle allant de $C_{12}$ à $C_{24}$, et peuvent être saturées ou non et/ou linéaires ou ramifiées.

- Les tensioactifs anioniques utilisables pour la présente invention peuvent être également des lipoamioacides, des dérivés alkylsulfoniques, et leurs mélanges.

Les lipoaminoacides peuvent être notamment choisis parmi les acylglutamates mono- et di-sodiques comme le sel disodique de l'acide N-stéaroyl L-glutamique commercialisé sous la dénomination ACYLGLUTAMATE HS21 par la société AJINOMOTO.

Les dérivés alkylsulfoniques peuvent être notamment choisis parmi les dérivés alkylsulfoniques de première formule suivante :

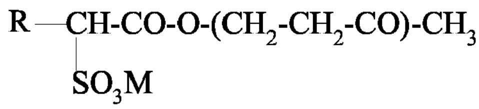

dans laquelle R représente un radical alkyle comportant de 16 à 22 atomes de carbone, et notamment un radical en $C_{16}H_{33}$ et $C_{18}H_{37}$ pris en mélange ou séparément, et M est un métal alcalin tel que le sodium, par exemple.

- Les tensioactifs cationiques convenant pour la préparation des particules et/ou dispersions conformes à l'invention peuvent être notamment choisis parmi les sels d'ammonium quaternaires, les amines grasses et leurs sels, et leurs mélanges.

Les sels d'ammonium quaternaires sont par exemple :

a) ceux qui présentent la seconde formule générale suivante :

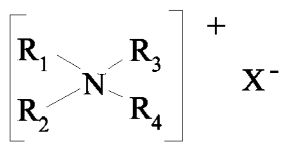

dans laquelle les radicaux $R_1$ à $R_4$, qui peuvent être identiques ou différents, représentent un radical aliphatique, linéaire ou ramifié, comportant de 1 à 30 atomes de carbone, ou un radical aromatique tel que aryle ou alkylaryle. Les radicaux aliphatiques peuvent comporter des hétéroatomes tels que notamment l'oxygène, l'azote, le soufre, les halogènes. Les radicaux aliphatiques sont par exemple choisis parmi les radicaux alkyle, alcoxy, polyoxyalkylène ($C_2$-$C_6$), alkylamide, alkyl($C_{12}$-$C_{22}$)amido alkyle($C_2$-$C_6$), alkyl($C_{12}$-$C_{22}$)acétate, hydroxyalkyle, comportant environ de 1 à 30 atomes de carbone ; X est un anion choisi dans le groupe des halogénures, phosphates, acétates, lactates, alkyl($C_2$-$C_6$)sulfates, alkyl-ou-alkylarylsulfonates.
Comme sels d'ammonium quaternaire présentant la seconde formule précédemment exposée, avantageusement utilisée, on peut mentionner d'une part, les chlorures de tétraalkylammonium comme par exemple les chlorures de dialkyldiméthylammonium ou d'alkyltriméthylammonium, dans lesquels le radical alkyle comporte environ de 12 à 22 atomes de carbone, en particulier les chlorures de béhényltriméthylammonium, de distéaryldiméthyl-ammonium, de cétyltriméthylammonium, de benzyl diméthyl stéaryl-ammonium ou encore, d'autre part, le chlorure de stéaramidopropyldiméthyl (myristyl acétate) ammonium commercialisé sous la dénomination "CERAPHYL 70" par la société VAN DYK.
b) les sels d'ammonium quaternaire de l'imidazolinium, comme par exemple ceux de troisième formule générale suivante :

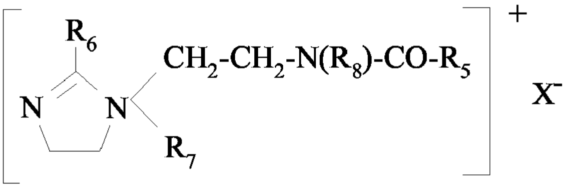

dans laquelle $R_5$ représente un radical alcényle ou alkyle comportant de 8 à 30 atomes de carbone, par exemple dérivé des acides gras du suif ; $R_6$ représente un atome d'hydrogène, un radical alkyle comportant de 1 à 4 atomes de carbone ou un radical alcényle ou alkyle comportant de 8 à 30 atomes de carbone ; $R_7$ représente un radical alkyle comportant de 1 à 4 atomes de carbone ; $R_8$ représente un atome d'hydrogène, un radical alkyle comportant de 1 à 4 atomes de carbone ; X est un anion choisi dans le groupe des halogénures, phosphates, acétates, lactates, alkylsulfates, alkyl-ou-alkylarylsulfonates.

En particulier, $R_5$ et $R_6$ désignent un mélange de radicaux alcényle ou alkyle comportant de 12 à 21 atomes de carbone, par exemple dérivés des acides gras du suif, $R_7$ désigne un radical méthyle, $R_8$ désigne l'hydrogène. Un tel produit est par exemple vendu sous la dénomination "REWOQUAT W 75" par la société REWO.

c) les sels de diammonium quaternaire de quatrième formule générale suivante

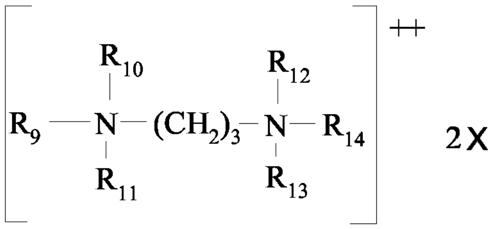

dans laquelle $R_9$ désigne un radical aliphatique comportant environ de 16 à 30 atomes de carbone ; $R_{10}$, $R_{11}$, $R_{12}$, $R_{13}$, et $R_{14}$ sont choisis parmi l'hydrogène ou un radical alkyle comportant de 1 à 4 atomes de carbone ; et X est un anion choisi dans le groupe des halogénures, acétates, phosphates, nitrates et méthylsulfates. De tels sels de diammonium quaternaire comprennent notamment le dichlorure de propanesuif diammonium.

A titre d'exemple d'agents tensioactifs convenant à la mise en oeuvre de l'invention, on peut citer le PEG-30 stéarate de glycéryle, le stéaroylglutamate de disodium, et leurs mélanges.

Selon encore une autre variante de réalisation, les dispersions conformes à la présente invention peuvent comprendre en tant que tensioactif non-ionique, un mélange de PEG-30 stéarate de glyceryle et de stéaroylglutamate de disodium.

La teneur en tensioactif non ionique et/ou tensioactif ionique, utilisés pour la préparation des particules et/ou des dispersions conformes à l'invention peut avantageusement varier de 0,5 et 50 % en poids, voire de 1 à 40 % en poids, et en particulier de 5 à 20 % en poids par rapport au poids total de la dispersion.

## POLYMÈRES ÉMULSIONNANTS

Les particules peuvent également contenir un polymère émulsionnant, c'est-à-dire un polymère amphiphile.

Parmi les polymères émulsionnants convenant à la mise en oeuvre de l'invention, il peut être fait mention :

- des copolymères di et tribloc POE-POP tels que ceux décrits dans le brevet US 6 464 990 ;
- des tensioactif siliconé et polyoxyéthyléné tels que ceux décrits dans le brevet US 6 120 778 ;
- des AMPS hydrophobés non réticulés tels que ceux décrits dans EP 1 466 588 ;
- des polymères acryliques amphiphiles, tels que les PEMULEN TR-1 ou TR-2 ou équivalent ;
- des polymères associatifs et gélifiants décrits dans US 2003/0138465 ;
- des polymères thermogélifiants tels que ceux décrits dans les demandes US 2004/0214913, US 2003/0147832 et US 2002/0198328 et FR 2 856 923.

Lorsqu'ils sont présents, le ou les polymères émulsionnants peuvent être introduits en une teneur variant de

0,1 à 15 % en poids, voire de 0,1 % à 10 % en poids, et plus en particulier de 0,1 à 5 % en poids par rapport au poids total de la dispersion.

**PROCEDE D'OBTENTION DES DISPERSIONS**

Les particules huileuses calibrées et sphériques, structurées, conformes à l'invention, peuvent être obtenues sous la forme d'une dispersion au moyen d'un procédé comprenant au moins les étapes consistant à :

- émulsifier un mélange d'au moins un filtre solaire, d'au moins une huile ou une phase huileuse et d'au moins un polymère gélifiant de la phase huileuse avec une phase aqueuse et/ou hydrosoluble à une température supérieure à la température de gélification du polymère,
- soumettre le mélange à un processus conduisant à l'obtention des particules huileuses, à une température d'au moins 5 à 10 °C supérieure à la température de fusion du mélange mis en oeuvre dans l'étape précédente, et
- refroidir la dispersion de particules ainsi obtenue.

On précise que la présence d'eau dans la première étape du procédé et la réalisation de la deuxième étape à chaud sont des conditions cumulatives nécessaires à l'obtention de particules calibrées sphériques selon l'invention.

La mesure de la viscosité est bien effectuée au niveau du mélange initial, à savoir du "macrogel" et non lorsque les particules sont déjà formées, comme cela a déjà été précisé plus haut.

Le procédé selon l'invention peut, le cas échéant, comprendre en outre une étape consistant à diluer la phase continue du mélange avant l'étape de refroidissement.

Au sens de la présente invention, on entend désigner par "processus conduisant à l'obtention des particules huileuses", une action de type cisaillement ou un mécanisme de type inversion de phase.

La température à laquelle est réalisée l'étape d'émulsification, est avantageusement supérieure à 40 °C, et avantageusement inférieure à 95 °C.

Ainsi, à l'issue du procédé, les dispersions conformes à l'invention comprennent dans une phase aqueuse et/ou hydrosoluble des particules huileuses calibrées comprenant au moins un filtre solaire et comprenant une phase huileuse structurée par au moins un polymère gélifiant.

La nature du processus exercé sur le mélange phase huileuse/polymère gélifiant détermine la taille des particules à obtenir.

Ainsi, pour les particules submicroniques, d'une taille moyenne d'environ 150 nm à 1 µm, on peut utiliser avantageusement des procédés développant un cisaillement turbulent tels que les ultrasons, l'homogénéisation à haute pression (pression d'usage entre 50 et 1000 bar) par exemple en utilisant le SOAVI OBL 20® de NIRO SOAVI, ou le MICROFLUIDIZER® de MICROFLUIDICS. On peut également utiliser les procédés ne nécessitant pas d'apport d'énergie mécanique tels que ceux mettant en jeu une inversion de phase lors de l'émulsification, comme la P.I.T (phase inversion temperature), l'inversion de composition (par exemple par ajout d'un tensio actif hydrophile dans une émulsion E/H pour l'inverser en H/E).

Pour les particules microniques, d'une taille moyenne d'environ 1 µm à 20 µm, on peut utiliser, par exemple, des procédés permettant d'obtenir la plus faible polydispersité possible tels que le cisaillement contrôlé d'émulsions viscoélastiques comme décrit dans la demande FR 2 747 321 et le brevet US 5 558 820, des procédés en continu tels que décrits dans le brevet US 5 688 842 et la demande WO 02/40574 ou ceux, plus généralement utilisant un moulin colloïdal, un mélangeur statique, un micromélangeur, une pale cadre, ou encore une membrane poreuse comme décrit dans le brevet US 5326484. On peut également utiliser des procédés faisant appel au contrôle du mûrissement (US 6 160 061), au gonflement d'un latex "templating agent" (EP 719 087), aux instabilités de Rayleigh (Weitz, Langmuir, 16, 347-351, (2000)) ou par fractionnement d'émulsions polydisperses (Bibette, J. Coll. Int. Sci., vol 147, n°2, 474-478, (1991)).

Afin de faciliter la formation des particules, lors de l'étape d'émulsification, on peut, par exemple, utiliser un ou des tensioactifs non-ioniques ou ioniques et/ou des polymères émulsionnants hydrophobes, tels que définis précédemment.

Par ailleurs, dans le cas par exemple de procédés développant un cisaillement laminaire pour obtenir une distribution de taille de particules homogène, il peut être, éventuellement, avantageux d'ajuster le rapport entre la viscosité de la phase huileuse dispersée et la viscosité de la phase aqueuse et/ou hydrophile continue dans un rapport allant de 0,01 à 5, voire de 0,05 à 2. Cet ajustement peut se faire, notamment, par l'addition de tensioactifs et/ou de polymère émulsionnant tels que ceux décrit précédemment et/ou de polymères gélifiants hydrophiles.

Ainsi, selon un mode de réalisation de l'invention, la phase continue aqueuse et/ou hydrosoluble peut, en outre, comprendre au moins un polymère hydrophile gélifiant.

Avantageusement, lorsqu'il est présent, le polymère hydrophile gélifiant est introduit dans la phase continue aqueuse, ou hydrophile, en une proportion allant de 0,01 % à 30 % en poids, notamment de 0,05 % à 15 % en poids

par rapport au poids total de la composition.

A titre d'exemple de polymères hydrophiles gélifiants, il peut être fait mention, notamment, des carbomers, de l'acrylamidométhylpropane sulfonique (AMPS), des dérivés de cellulose ou de guar. A titre de dérivés de guar susceptibles d'être avantageusement utilisés dans la mise en oeuvre de la présente invention, il est possible de citer l'hydroxypropylguar commercialisé sous la référence JAGUAR HP® 105 par la société RHODIA.

Ainsi, le mélange phase huileuse - phase aqueuse et/ou hydrosoluble peut comprendre, en outre, un composé choisi parmi un agent tensioactif, un polymère émulsionnant, un polymère gélifiant hydrophile, et leurs mélanges, et de préférence un mélange d'un agent tensioactif, d'un polymère émulsionnant et d'un polymère gélifiant hydrophile.

Ainsi, en raison du procédé de préparation des particules conformes à l'invention, les particules conformes à la présente invention sont avantageusement dépourvues de solvant volatile.

## DISPERSION

Conformément au procédé d'obtention des particules conformes à l'invention, tel que décrit précédemment, ces dernières sont obtenues en dispersion dans une phase continue et/ou hydrosoluble.

La teneur en particules huileuses calibrées, comprenant au moins un filtre solaire et comprenant une phase huileuse structurée par un polymère gélifiant, selon l'invention présentes dans la phase continue aqueuse et/ou hydrosoluble peut notamment être telle que la fraction massique huileuse dispersée dans la phase aqueuse et/ou hydrosoluble peut varier de 5 à 89 % en poids, notamment de 20 à 85 % en poids, voire de 40 à 80 % en poids, et en particulier de 60 à 80 % en poids par rapport au poids total de la dispersion.

Les particules huileuses calibrées, structurées, conformes à l'invention, avantageusement ne s'agrègent pas dans la dispersion dans laquelle elles sont obtenues, et leurs spécificités granulométriques en terme de taille et d'indice de distribution y sont avantageusement conservées.

La phase continue aqueuse et/ou hydrosoluble convenant à la mise en oeuvre de l'invention peut être, avantageusement, de l'eau et/ou un solvant organique hydrosoluble, comme par exemple des glycols comme la glycérine, le dipropylène glycol, seuls ou en mélanges.

On entend désigner par l'expression "solvant hydrosoluble", au sens de la présente invention, un composé liquide à température ambiante et miscible à l'eau (miscibilité dans l'eau supérieure à 50 % en poids à 25 °C, et à pression atmosphérique).

Parmi les solvants hydrosolubles pouvant être utilisés dans les dispersions conformes à l'invention, on peut mentionner, notamment les monoalcools inférieurs ayant de 1 à 5 atomes de carbone, tels que l'éthanol et l'isopropanol, les glycols ayant de 2 à 8 atomes de carbone, tels que l'éthylène glycol, le propylène glycol, le 1,3-buthylèneglycol et le dipropylèneglycol, les cétones en $C_3$ et $C_4$, la glycérine et les aldéhydes en $C_2$-$C_4$.

Selon encore une autre variante de réalisation, les dispersions conformes à la présente invention peuvent comprendre en tant que phase aqueuse continue, de l'eau déminéralisée.

Selon encore une autre variante de réalisation, les dispersions conformes à l'invention peuvent comprendre en tant que tensioactif non-ionique un mélange de PEG-30 stéarate de glyceryle et de stéaroylglutamate de disodium, et en tant que polymère hydrophile gélifiant, de l'hydroxypropyle guar.

Selon encore une autre variante de réalisation, la phase continue aqueuse et/ou hydrosoluble convenant à la mise en oeuvre de l'invention peut contenir des agents photoprotecteurs complémentaires hydrophiles actifs dans l'UV-A et ou l'UV-B.

Parmi les filtres UV organiques hydrophiles utilisables selon l'invention, on peut citer ceux désignés ci-dessus sous leur nom INCI :

(1) les dérivés de p-aminobenzoïque (PABA) comme

- PABA,
- Glyceryl PABA, et
- PEG-25 PABA vendu sous le nom "UVINUL P25" par BASF.

(2) les dérivés de benzophénone comportant au moins un radical sulfonique comme

- Benzophenone-4 vendu sous le nom commercial " UVINUL MS40" par BASF,
- Benzophenone-5, et
- Benzophenone-9.

(3) les dérivés de benzylidène camphre comportant au moins un radical sulfonique comme par exemple :

- Benzylidène Camphre Acide Sulfonique fabriqué sous le nom "MEXORYL SL" par CHIMEX,
- Camphre Benzalkonium Methosulfate fabriqué sous le nom "MEXORYL SO" par CHIMEX, et
- Terephthalylidène Dicamphre Acide Sulfonique fabriqué sous le nom "MEXORYL SX" par CHIMEX.

(4) les dérivés de benzimidazole comportant au moins un radical sulfonique comme par exemple :

- Phenylbenzimidazole Sulfonic Acid vendu notamment sous le nom commercial "EUSOLEX 232" par MERCK,
- les dérivés bis-benzoazolyle tels que décrits dans les brevets EP 669 323, et US 2,463,264 et plus particulièrement le composé Disodium Phenyl Dibenzimidazole Tetra-sulfonate vendu sous le nom commercial commercial "NEO HELIOPAN AP" par Haarmann et REIMER,

(5) les dérivés de cinnamate hydrophiles comme par exemple le DEA Methoxycinnamate, et

(6) leurs mélanges.

Parmi ces filtres hydrophiles, les plus préférentiels sont choisis parmi

- Terephthalylidène Dicamphre Acide Sulfonique,
- Benzophenone-4,
- Phenylbenzimidazole Sulfonic Acid,
- Disodium Phenyl Dibenzimidazole Tetra-sulfonate,
- ainsi que leurs mélanges.

## COMPOSITION COSMETIQUE OU DERMATOLOGIQUE

Les particules et/ou les dispersions conformes à l'invention, comprenant au moins un filtre solaire peuvent être, avantageusement, introduites dans différentes formulations cosmétiques et/ou dermatologiques pour une application topique sur la peau et plus particulièrement destinées à la photoprotection.

Ainsi, les particules et/ou les dispersions conformes à la présente invention peuvent être utilisées pour la préparation de composition(s) cosmétique(s) ou dermatologique(s) utilisable(s) dans le domaine de la photoprotection des matières kératiniques et plus particulièrement de la peau et des cheveux.

Ainsi, la présente invention a encore pour objet des compositions cosmétiques ou dermatologiques comprenant au moins des particules et/ou au moins une dispersion telle que précédemment définies. Ces compositions sont utiles comme produit pour la protection des matières kératiniques contre les rayonnements UV et en particulier la peau.

Les compositions comportant des particules et/ou des dispersions conformes à l'invention peuvent être des compositions pour le soin, l'hygiène et/ou le maquillage, notamment de la peau et des phanères.

En l'occurrence, une composition selon l'invention peut se présenter sous forme de produits de maquillage tels que des mascaras, des produits pour les sourcils, des eyeliners, des fards à paupières, des fards à joues, des fonds de teint, des produits pour les lèvres, des produits de maquillage du corps ou des cheveux et des produits capillaires tels que des shampoings, des après-shampooings, des lotions ou des gels.

Avantageusement, la composition peut contenir de 0,01 à 40 % en poids, notamment de 0,1 à 25 % en poids voire de 0,2 à 20 % en poids, de particules conformes à la présente invention par rapport au poids total de la composition.

La composition cosmétique ou dermatologique peut se présenter sous la forme de lotion, d'émulsion huile-dans-eau (H/E) ou eau-dans-huile (E/H) ou multiple (E/H/E), de gel aqueux ou hydroalcoolique, de crème, lait...

## ADDITIFS

Les compositions cosmétiques conformes à l'invention peuvent également comporter tout additif usuellement utilisé dans le domaine concerné sous réserve que ces additifs n'altèrent pas la propriété des compositions augmenter le facteur de protection solaire.

Dans le cadre des compositions destinées notamment au maquillage, les additifs susceptibles de convenir à la mise en oeuvre de l'invention peuvent être choisis notamment parmi des matières colorantes, comme les nacres et les pigments, des charges, des antioxydants, des agents filmogènes, et le cas échéant des auxiliaires de filmification, des huiles essentielles, des conservateurs, des parfums, des agents hydratants, des agents antiseptiques, des neutralisants, et leurs mélanges.

Bien entendu l'homme du métier veillera également à choisir les éventuels additifs complémentaires et/ou leur quantité de telle manière que, les propriétés avantageuses de la composition selon l'invention, ne soient pas ou substantiellement pas, altérées par l'adjonction envisagée.

La présente invention a encore pour objet un procédé cosmétique de maquillage et/ou de soin non thérapeutique, comprenant au moins l'étape d'application sur la peau d'une composition telle que définie précédemment.
La présente invention a enfin pour objet l'utilisation de particules et/ou d'au moins une dispersion conformes à l'invention pour la fabrication d'une composition destinée à la protection de la peau et/ou des cheveux contre les effets néfastes des rayonnements UV, en particulier le rayonnement solaire.
Les exemples de dispersions de particules et de compositions présentés ci-après sont donnés à titre illustratif et sans caractère limitatif de l'invention.

## EXEMPLES

Exemples 1 à 6 : matériaux contenant des filtres solaires:

| | 1 | 2 | 3 | 4 | 5 | 6 (comparatif) |
|---|---|---|---|---|---|---|
| **Filtres** | | | | | | |
| Butyl methoxydibenzoylmethane[1] | 13,1 % | 10,1 % | 10,8% | 10,1 % | 10,1 % | 13,1 % |
| Octocrylene[2] | 35,6 % | 27,4 % | 29,2 % | 27,4 % | 27,4% | 35,6 % |
| **Huiles** | | | | | | |
| Dicaprylyl carbonate[3] | 26,3% | | 40 % | | | 51,3% |
| isopropyl lauroylsarcosinate[4] | | 37,5% | | | 37,5% | |
| Bis-hydroxyethoxypropyl dimethicone[5] | | | | 37,5 % | | |
| **Polymère** | | | | | | |
| Ethylenediamine/stearyl DimerDilinoleate copolymer[6] | 25% | 25% | | | 12,5% | |
| Poly C10-30 alkyl acrylate[7] | | | 20 % | | | |
| Nylon-611/dimethicone copolymer (and) PPG-3 myristyl ether[8] | | | | 25% | 12,5% | |
| 1 : Parsol 1789 de la société Givaudan<br>2 : Uvinul N539 de la Société BASF<br>3 : Cetiol CC de la Société Cognis<br>4 : Eldew SL-205 de la Société Ajinomoto<br>5 : DC2-5562 Fluid de la Société Dow Coming<br>6 : Uniclear 100VG de la Société Arizona Chemical<br>7 : Doresco IPA 13-6 de la Société Landec Corporation<br>8 : DC2-8178 Gellant de la Société Dow Coming | | | | | | |

## Préparation des matériaux

Le mélange de filtre est homogénéisé si nécessaire à 80 °C. D'autre part on solubilise sous agitation, à chaud, le polymère dans l'huile, ce mélange est ensuite additionné au mélange de filtre et homogénéisé jusqu'à complète solubilisation.
Le mélange obtenu peut être refroidit à température ambiante et stocké ou utilisé directement pour la fabrication de microparticules.
**Exemple 7 :** mise en dispersion du matériau de l'exemple 1 sous forme de microparticules.

| | |
|---|---|
| Matériau obtenu selon l'exemple 1 | 40,0 % |
| PEG-30 Glyceryl Stearate (Tagat S de la Société Degussa) | 1,6 % |
| Disodium Stearoylglutamate (Amisoft HS21P de la Société Ajinomoto) | 0,4 % |
| Eau déminéralisée | 58,0 % |

La dispersion est réalisée dans un bécher de diamètre interne 72 mm comportant une double enveloppe permettant de réguler sa température par l'intermédiaire d'un bain chauffant. On y introduit une solution aqueuse composée de 6,4 g de PEG-30 Glyceryl Stearate, 1,6g de Disodium Stearoylglutamate et 32 g d'eau déminéralisée. Le mélange est porté à 85 °C. Par ailleurs on prépare 160 g du mélange correspondant au matériau 1 de l'exemple 1, que l'on porte à une température de 90 °C. Celui-ci est introduit en 10 minutes dans la solution aqueuse de tensioactif maintenue sous agitation par une pale de largeur 70 mm et d'épaisseur 2 mm avec une vitesse de rotation de 160 rpm. Après 3 minutes la vitesse de rotation est portée à 400 rpm et maintenue pendant 30 minutes.

L'émulsion est ensuite diluée avec 200 g d'eau déminéralisée à 80 °C pour atteindre une fraction massique en phase dispersée de 40 %, puis refroidie à température ambiante.

On obtient une dispersion homogène et stable de microparticules avec une granulométrie de 1,36 μm (d[v, 0.5]) et un facteur d'uniformité (U) de 0,24.

**Exemple 8 (comparatif) :** émulsion H/E obtenue avec le matériau de l'exemple 6.

En appliquant le mode opératoire de l'exemple 7, à l'exception de la vitesse de rotation de la pale qui est de 800 rpm, on obtient une émulsion homogène et stable avec une granulométrie de 1,63 μm (d[v,0.5]) et un facteur d'uniformité (U) de 0,22.

**Exemple 9 :** dispersion de microparticules obtenue à partir du matériau de l'exemple 2.

En appliquant le mode opératoire de l'exemple 7 on obtient une dispersion homogène et stable de microparticules avec une granulométrie de 1,07 μm (d[v,0.5]) et un facteur d'uniformité (U) de 0,24.

**Exemple 10 :** dispersion de microparticules obtenue à partir du matériau de l'exemple 3.

En appliquant le mode opératoire de l'exemple 7 on obtient une dispersion homogène et stable de microparticules avec une granulométrie de 2,46 μm (d[v,0.5]) et un facteur d'uniformité (U) de 0,42.

**Exemple 11 :** dispersion de microparticules obtenue à partir du matériau de l'exemple 4.

En appliquant le mode opératoire de l'Exemple 7 on obtient une dispersion homogène et stable de microparticules avec une granulométrie de 2,00 μm (d[v,0.5]) et un facteur d'uniformité (U) de 0,38.

**Exemple 12 :** mise en dispersion du matériau de l'exemple 1 sous forme de microparticules.

| | |
|---|---|
| Matériau obtenu selon l'exemple 1 | 40,0 % |
| PEG-30 Glyceryl Stearate (Tagat S de la Société Degussa) | 4,3 % |
| Disodium Stearoylglutamate (Amisoft HS21P de la Société Ajinomoto) | 1,1 % |
| Eau déminéralisée | 54,6 % |

La dispersion est réalisée dans un bécher de diamètre interne 72 mm comportant une double enveloppe permettant de réguler sa température par l'intermédiaire d'un bain chauffant. On y introduit une solution aqueuse composée de 12,8 g de PEG-30 Glyceryl Stearate, 3,2 g de Disodium Stearoylglutamate et 64g d'eau déminéralisée. Le mélange est porté à 85 °C. Par ailleurs on prépare 120 g du mélange correspondant au matériau de l'exemple 1, que l'on porte à une température de 90 °C. Celui-ci est introduit en 10 minutes dans la solution aqueuse de tensio-actif maintenue sous agitation par une pale de largeur 70 mm et d'épaisseur 2 mm avec une vitesse de rotation de 160 rpm. Après 3 minutes la vitesse de rotation est portée à 250 rpm et maintenue pendant 30 minutes.

L'émulsion est ensuite diluée avec 100g d'eau déminéralisée à 80 °C pour atteindre une fraction massique en phase dispersée de 40 %, puis refroidie à température ambiante.

On obtient une dispersion homogène et stable de microparticules avec une granulométrie de 8,97 μm (d[v, 0.5]) et un facteur d'uniformité (U) de 0,35.

**Exemple 13 :** microparticules contenant des filtres solaires

La dispersion est réalisée dans un bécher de diamètre interne 72 mm comportant une double enveloppe permettant de réguler sa température par l'intermédiaire d'un bain chauffant. On y introduit une solution aqueuse composée de 1,6 g de PEG-30 Glyceryl Stearate, 0,1 g de Disodium Stearoylglutamate, 2,3g d' Hydroxyethylcellulose et 116 g d'eau déminéralisée. Le mélange est porté à 85 °C. Par ailleurs on prépare 80 g du mélange suivant : 24 g de Ethylenediamine/stearyl DimerDilinoleate copolymer (Uniclear 100VG de la Société Arizona Chemical), 19,6 g de Dicaprylyl carbonate (Cetiol CC de la Société Cognis), 26,6 g d' Octocrylene (Uvinul N539 de la Société BASF) et 9,8 g de Butyl methoxydibenzoylmethane (Parsol 1789 de la société Givaudan), que l'on porte à une température de 90 °C.

Celui-ci est introduit en 10 minutes dans la solution aqueuse de tensio-actif maintenue sous agitation par une pale de largeur 70 mm et d'épaisseur 2 mm avec une vitesse de rotation de 300 rpm. Après 3 minutes la vitesse de rotation est portée à 600 rpm et maintenue pendant 30 minutes.

L'émulsion est ensuite refroidie à température ambiante.

On obtient une dispersion homogène et stable de microparticules avec la composition suivante :

| | |
|---|---|
| Filtres solaires gélifiés | 40,00 % |

(suite)

| | |
|---|---|
| PEG-30 Glyceryl Stearate (Tagat S de la Société Degussa) | 0,80 % |
| Disodium Stearoylglutamate (Amisoft HS21P de la Société Ajinomoto) | 0,05 % |
| Hydroxyethylcellulose (Natrosol 250HHR de la Société Aqualon) | 1,15% |
| Eau déminéralisée | 58,00 % |

La dispersion présente une granulométrie de 11,3 μm (d[v,0.5]) et un facteur d'uniformité (U) de 0,30.

**Exemple 14 :** microparticules contenant des filtres solaires

La dispersion est réalisée dans un bécher de diamètre interne 72 mm comportant une double enveloppe permettant de réguler sa température par l'intermédiaire d'un bain chauffant. On y introduit une solution aqueuse composée de 2,28 g de PEG-30 Glyceryl Stearate, 0,12 g de Disodium Stearoylglutamate, 1,8 g d' HydroxyPropyl Guar (Jaguar HP105 de la Société Rhodia) et 115,8 g d'eau déminéralisée. Le mélange est porté à 85 °C. Par ailleurs on prépare 80 g du matériau de l'exemple 1 que l'on porte à une température de 90 °C.
Celui-ci est introduit en 10 minutes dans la solution aqueuse de tensio-actif maintenue sous agitation par une pale de largeur 70 mm et d'épaisseur 2 mm avec une vitesse de rotation de 300 rpm. Après 3 minutes la vitesse de rotation est portée à 600 rpm et maintenue pendant 30 minutes.
L'émulsion est ensuite refroidie à température ambiante.
On obtient une dispersion homogène et stable de microparticules avec la composition suivante :

| | |
|---|---|
| Filtres solaires gélifiés | 40,00 % |
| PEG-30 Glyceryl Stearate (Tagat S de la Société Degussa) | 1,14% |
| Disodium Stearoylglutamate (Amisoft HS21P de la Société Ajinomoto) | 0,06 % |
| HydroxyPropyl guar (Jaguar HP105 de la Société Rhodia) | 0,90 % |
| Eau déminéralisée | 57,90 % |

La dispersion présente une granulométrie de 7,4 μm (d[v,0.5]) et un facteur d'uniformité (U) de 0,40.

**Exemple 15 :** microparticules contenant des filtres solaires

La dispersion est réalisée dans un bécher de diamètre interne 72 mm comportant une double enveloppe permettant de réguler sa température par l'intermédiaire d'un bain chauffant. On y introduit une solution aqueuse composée de 12 g d'alcool polyvinylique (Celvol 203 de la Société CELANESE) et 68 g d'eau déminéralisée. Le mélange est porté à 85 °C. Par ailleurs on prépare 120 g du matériau de l'exemple 1 que l'on porte à une température de 90 °C. Celui-ci est introduit en 10 minutes dans la solution aqueuse de tensioactif maintenue sous agitation par une pale de largeur 70 mm et d'épaisseur 2 mm avec une vitesse de rotation de 160 rpm. Après 3 minutes la vitesse de rotation est portée à 400 rpm et maintenue pendant 30 minutes.
L'émulsion est ensuite refroidie à température ambiante.
On obtient une dispersion homogène et stable de microparticules avec la composition suivante :

| | |
|---|---|
| Filtres solaires gélifiés | 40,00 % |
| Alcool polyvinylique (Celvol 203 de la Société Celanese) | 4,00 % |
| Eau déminéralisée | 56,00 % |

La dispersion présente une granulométrie de 6,85 μm (d[v,0.5]) et un facteur d'uniformité (U) de 0,40.

**Exemple 16 :** nanoparticules contenant des filtres solaires

| | |
|---|---|
| Matériau obtenu selon l'exemple 2 | 20,00 % |
| PEG-30 Glyceryl Stearate (Tagat S de la Société Degussa) | 1,80 % |
| Disodium Stearoylglutamate (Amisoft HS21P de la Société Ajinomoto) | 0,20 % |

(suite)

| | |
|---|---|
| Eau déminéralisée | 78,00 % |

On prépare une solution aqueuse composée de 9 g de PEG-30 Glyceryl Stearate, 1 g de Disodium Stearoylglutamate et 390 g d'eau déminéralisée. Le mélange est porté à 85 °C. Par ailleurs on prépare 100 g du mélange correspondant au matériau 2, que l'on porte à une température de 90 °C. Celui-ci est introduit dans la solution aqueuse de tensioactif maintenue sous agitation par un dispositif de type rotor-stator (UltraTurrax T50). Après 5 minutes le mélange est homogénéisé à 85 °C à l'aide d'un homogénéisateur Soavi Panda, en 2 passages à une pression de 400 bar.
La dispersion est ensuite refroidie à température ambiante.
On obtient une dispersion homogène et stable de nanoparticules avec un diamètre moyen de 300 nm.
On compare les SPF (in vitro) des compositions suivantes :

**<u>Exemple 17 :</u>**

| | |
|---|---|
| Émulsion de l'exemple 8 | 50,00 % |
| HydroxyPropyl guar (Jaguar HP105 de la Société Rhodia) | 0,50 % |
| Eau déminéralisée | 49,50 % |

**<u>Exemple 18 :</u>**

| | |
|---|---|
| Dispersion de l'exemple 7 | 50,00 % |
| HydroxyPropyl guar (Jaguar HP105 de la Société Rhodia) | 0,50 % |
| Eau déminéralisée | 49,50 % |

Le facteur de protection solaire (SPF) est déterminé selon la méthode *"in vitro"* décrite par B.L. Diffey dans J. Soc. Cosmet. Chem. 40 , 127-133, (1989).
Les mesures ont été réalisées à l'aide d'un spectrophotomètre UV-1000S de la société Labsphere. Chaque composition est appliquée sur un ruban adhésif TRANSPORE de chez 3M collé sur une lame de quartz, sous la forme d'un dépôt homogène et régulier à raison de 1 mg/cm2.

| | SPF | erreur |
|---|---|---|
| Exemple 17 | 6,9 | 0,13 |
| Exemple 18 | 32,2 | 0,29 |

On constate que les particules de l'invention permettent d'améliorer le facteur de protection solaire comparativement à une émulsion de même granulométrie ne contenant pas de polymère cristallin.
On compare également les SPF des compositions suivantes :

**<u>Exemple 19 (comparatif) :</u>** émulsion H/E sans polymère cristallin

| | |
|---|---|
| <u>Phase A</u> | |
| PEG-20 methyl glucose sesquistearate | |
| (GLUCAMATE SSE 20 de Chemron) | 2,00 % |
| Disodium EDTA | 0,10 % |
| Glycerin | 5,00 % |
| Conservateur | 0,20 % |
| Eau déminéralisée | 56,70 % |

Phase B

| | |
|---|---|
| Methyl glucose sesquistearate | 2,00 % |
| Alcool stéarique (et) Ceteareth-20 | 2,00 % |
| Cyclohexasiloxane | 5,85 % |
| C12-15 alkyl benzoate | 5,85 % |
| Dicaprylyl carbonate | 2,62 % |
| Butyl methoxydibenzoylmethane | 1,32 % |
| Octocrylene | 3,56 % |
| Ethylenediamine/stearyl DimerDilinoleate copolymer (Uniclear 100VG de la Société Arizona Chemical) | 2,50 % |
| Conservateur | 0,10 % |

Phase C

| | |
|---|---|
| Polyacrylamide (and) C13-14 isoparaffin (and) Laureth-7 (Sepigel 305 de Seppic) | 1,00 % |
| Ammonium Polyacryloyldimethyl Taurate (Hostacerin AMPS de Clariant) | 1,20 % |

Phase D

| | |
|---|---|
| Biosaccharide Gum-1 (Fucogel 1000PP de Solabia) | 5,00 % |

Phase E

| | |
|---|---|
| Aluminium Starch Octenylsuccinate | 3,00 % |

**Exemple 20 (comparatif) :** émulsion H/E avec polymère cristallin

Phase A

| | |
|---|---|
| PEG-20 methyl glucose sesquistearate (GLUCAMATE SSE 20 de Chemron) | 2,00 % |
| Disodium EDTA | 0,10 % |
| Glycerin | 5,00 % |
| Conservateur | 0,20 % |
| Eau déminéralisée | 56,70 % |

Phase B

| | |
|---|---|
| Methyl glucose sesquistearate | 2,00 % |
| Alcool stéarique (et) Ceteareth-20 | 2,00 % |
| Cyclohexasiloxane | 7,10 % |
| C 12-15 alkyl benzoate | 7,10 % |
| Dicaprylyl carbonate | 2,62 % |
| Butyl methoxydibenzoylmethane | 1,32 % |
| Octocrylene | 3,56 % |
| Conservateur | 0,10 % |

Phase C

| | |
|---|---|
| Polyacrylamide (and) C13-14 isoparaffin (and) Laureth-7 (Sepigel 305 de Seppic) | 1,00 % |
| Ammonium Polyacryloyldimethyl Taurate (Hostacerin AMPS de Clariant) | 1,20 % |

Phase D
Biosaccharide Gum-1 (Fucogel 1000PP de Solabia) 5,00 %
Phase E
Aluminum Octenylsuccinate amidoné 3,00 %

**Exemple 21** : émulsion H/E contenant les microparticules de l'invention

Phase A

| | |
|---|---|
| PEG-20 methyl glucose sesquistearate (GLUCAMATE SSE 20 de Chemron) | 2,00 % |
| Disodium EDTA | 0,10 % |
| Glycerin | 5,00 % |
| Conservateur | 0,20 % |
| Eau déminéralisée | 41,70 % |

Phase B

| | |
|---|---|
| Methyl glucose sesquistearate | 2,00 % |
| Alcool stéarique (et) Ceteareth-20 | 2,00 % |
| Cyclohexasiloxane | 5,85 % |
| $C_{12-15}$ alkyl benzoate | 5,85 % |
| Conservateur | 0,10 % |

Phase C

| | |
|---|---|
| Polyacrylamide (and) C13-14 isoparaffin (and) Laureth-7 (Sepigel 305 de Seppic) | 1,00 % |
| Ammonium Polyacryloyldimethyl Taurate (Hostacerin AMPS de Clariant) | 1,20 % |

Phase D
Biosaccharide Gum-1 (Fucogel 1000PP de Solabia) 5,00 %

Phase E
Aluminum Octenylsuccinate amidoné 3,00 %

Phase F
Dispersion de microparticules de l'exemple 7 25,00 %

Mesures de SPF

| | SPF | erreur |
|---|---|---|
| Exemple 19 (comparatif) | 10,4 | 0,24 |
| Exemple 20 (comparatif) | 12,2 | 0,19 |
| Exemple 21 | 22,8 | 0,18 |

Au vu de ces résultats on observe une augmentation du SPF lorsque l'on introduit un gélifiant des huiles dans la phase grasse d'une émulsion. De plus cette augmentation de SPF est potentialisée lorsque les filtres solaires sont

introduits dans l'émulsion sous forme de microparticules calibrées selon l'invention.

## Revendications

1. Particules huileuses calibrées et sphériques comprenant au moins un filtre solaire hydrophobe et comprenant au moins une phase huileuse structurée par au moins un polymère gélifiant, **caractérisées en ce qu'**elles possèdent une taille moyenne inférieure ou égale à 20 µm, **en ce que** ladite phase huileuse structurée possède un point de fusion supérieur ou égal à 40 °C et **en ce que** leur indice de circularité est compris entre 0,9 et 1.

2. Particules huileuses calibrées et sphériques comprenant au moins un filtre solaire et comprenant au moins une phase huileuse structurée par au moins un polymère gélifiant, **caractérisée en ce qu'**elles possèdent une taille moyenne inférieure ou égale à 20 µm, **en ce que** ledit polymère gélifiant est choisi parmi un polymère semi-cristallin, un polyamide, un polyamide siliconé, un monoalkylester ou un polyalkylester de polysaccharide, un copolymère dibloc et/ou tribloc et/ou multibloc et/ou radial-bloc, et leurs mélanges, et **en ce que** leur indice de circularité est compris entre 0,9 et 1.

3. Particules huileuses calibrées et sphériques comprenant au moins un filtre solaire, **caractérisées en ce qu'**elles sont obtenues à partir d'une phase huileuse structurée par au moins un polymère gélifiant, ledit polymère gélifiant étant de nature et/ou à une teneur suffisante pour conférer à ladite phase huileuse une viscosité supérieure ou égale à 750 Pa.s au cisaillement de 1 $s^{-1}$, à 25 °C et **en ce que** leur indice de circularité est compris entre 0,9 et 1.

4. Particules selon l'une quelconque des revendications précédentes, **caractérisées en ce qu'**elles possèdent une taille moyenne inférieure ou égale à 20 µm, en particulier inférieure ou égale à 15 µm, et plus particulièrement inférieure ou égale à 12 µm.

5. Particules selon la revendication précédente, **caractérisées en ce qu'**elles possèdent une taille moyenne variant de 100 nm à 20 µm, voire allant de 100 nm à 15 µm, et en particulier allant de 150 nm à 12 µm.

6. Particules selon l'une quelconque des revendications précédentes, **caractérisées en ce que** la phase huileuse structurée possède un point de fusion inférieur ou égal à 95 °C, de préférence variant de 50 à 90 °C.

7. Particules selon l'une quelconque des revendications précédentes, **caractérisées en ce que**, lorsqu'elles ont une taille moyenne micrométrique, elles possèdent un coefficient d'uniformité inférieur ou égal à 0,45.

8. Particules selon l'une quelconque des revendications 1 à 5, **caractérisées en ce que**, lorsqu'elles ont une taille moyenne submicrométrique, elles possèdent un indice de polydispersité inférieur ou égal à 0,35.

9. Particules selon l'une quelconque des revendications précédentes, **caractérisées en ce que** leur forme est substantiellement sphérique.

10. Particules selon l'une quelconque des revendications précédentes, **caractérisées en ce que** la phase huileuse comprend au moins une huile non volatile.

11. Particules selon l'une quelconque des revendications précédentes, **caractérisées en ce que** la phase huileuse est présente en une teneur variant de 10 à 99 % en poids, en particulier de 15 à 99 % en poids, plus particulièrement de 20 à 99 % en poids par rapport au poids total de la particule.

12. Particules selon l'une quelconque des revendications précédentes, **caractérisées en ce que** la phase huileuse comprend au moins une huile choisie parmi une huile végétale, une huile animale, une huile synthétique, une huile minérale et leurs mélanges.

13. Particules selon l'une quelconque des revendications 1, 3 à 12, **caractérisées en ce que** le polymère gélifiant est choisi parmi un polymère semi-cristallin, un polyamide, un polyamide siliconé, un monoalkylester de polysaccharide, un polyalkylester de polysaccharide, un copolymère dibloc et/ou tribloc et/ou multibloc et/ou radial-bloc, et leurs mélanges.

14. Particules selon l'une quelconque des revendications précédentes, **caractérisées en ce que** le polymère gélifiant

est choisi parmi l'éthylènediamine/stéaryle dimerdilinoléate, le polymère semi-cristallin de type poly-$C_{10-30}$ alkylacrylate, et leurs mélanges.

**15.** Particules selon l'une quelconque des revendications précédentes, **caractérisées en ce que** le polymère gélifiant est présent en une teneur variant de 1 à 80 % en poids, voire de 1 à 60 % en poids par rapport au poids total de la particule.

**16.** Particules selon l'une quelconque des revendications précédentes, **caractérisées en ce que** le rapport pondéral entre le polymère gélifiant et la phase huileuse de la particule varie de 0,01 à 4, voire de 0,05 à 2, et en particulier de 0,1 à 1.

**17.** Particules selon l'une quelconque des revendications précédentes, **caractérisées en ce que** le filtre solaire hydrophobe est choisi parmi les agents photoprotecteurs organiques hydrophobes, les agents photoprotecteurs inorganiques et leurs mélanges.

**18.** Particules selon l'une quelconque des revendications précédentes, **caractérisées en ce que** le filtre solaire hydrophobe est choisi parmi les anthranilates, les dérivés cinnamiques, les dérivés de dibenzoylméthane, les dérivés salyciliques, les dérivés du camphre, les dérivés de triazine, les dérivés de la benzophénone, les dérivés de β,β-diphénylacrylate, les dérivés de benzotriazole, les dérivés de benzalmalonate, les dérivés de benzimidazole, les imidazolines, les dérivés bis-benzoazolyle, les dérivés de l'acide p-aminobenzoïque (PABA), les dérivés de méthylène bis-(hydorxyphényl benzotriazole), les polymères filtres et silicones filtres, les dimères dérivés d'a-alkylstyrène, les 4,4-diarylbutadiènes et leurs mélanges.

**19.** Particules selon la revendication 17 ou 18, **caractérisées en ce que** le filtre solaire est présent en une teneur variant de 0,05 à 80 % en poids, de 0,1 à 60 % en poids, et en particulier de 0,5 à 50 % en poids par rapport au poids total de la particule.

**20.** Dispersion en phase aqueuse et/ou hydrosoluble comprenant des particules selon l'une quelconque des revendications 1 à 19.

**21.** Dispersion selon la revendication précédente, **caractérisée en ce qu'**elle comprend en outre au moins un agent tensioactif choisi parmi un agent tensioactif ionique, un agent tensioactif non ionique, et leurs mélanges.

**22.** Dispersion selon l'une quelconque des revendications 20 ou 21, **caractérisée en ce qu'**elle comprend en outre au moins un polymère émulsionnant hydrophobe.

**23.** Dispersion selon l'une quelconque des revendications 20 à 22, **caractérisée en ce qu'**elle comprend en outre au moins un polymère hydrophile gélifiant.

**24.** Dispersion selon l'une quelconque des revendications 20 à 23, **caractérisée en ce que** les particules d'huiles structurées dispersées dans la phase aqueuse représentent une fraction massique huileuse variant de 5 à 89 % en poids par rapport au poids total de la dispersion, de préférence de 20 à 85 % en poids, voire de 40 à 80 % en poids, et en particulier de 60 à 80 % en poids.

**25.** Composition cosmétique ou dermatologique comprenant au moins des particules telles que définies selon l'une quelconque des revendications 1 à 19, et/ou au moins une dispersion telle que définie selon l'une quelconque des revendications 20 à 24.

**26.** Procédé de fabrication d'une dispersion de particules huileuses calibrées et sphériques comprenant au moins un filtre solaire et au moins une phase huileuse structurée par au moins un polymère gélifiant et possédant une taille moyenne inférieure ou égale à 20 μm, comprenant au moins les étapes consistant à :

- émulsifier un mélange d'au moins un filtre solaire, d'au moins une huile ou phase huileuse et d'au moins un polymère gélifiant de ladite phase huileuse avec une phase aqueuse et/ou hydrosoluble, à une température supérieure à la température de gélification dudit polymère,
- soumettre le mélange à un processus conduisant à obtenir lesdites particules huileuses, à une température d'au moins 5 à 10 °C supérieure à la température de fusion du mélange mis en oeuvre dans l'étape précédente, et
- refroidir la dispersion des particules ainsi obtenues.

**27.** Procédé selon la revendication précédente, **caractérisé en ce que** ledit polymère gélifiant est tel que défini en revendications 13 à 15.

**28.** Procédé selon la revendication 26 ou 27, **caractérisé en ce que** ledit processus conduisant à obtenir des particules est adapté à l'obtention de particules d'une taille moyenne allant d'environ 1 à environ 15 micromètres.

**29.** Procédé selon la revendication précédente, **caractérisé en ce que** ledit processus est choisi parmi le cisaillement contrôlé d'émulsions viscoélastiques, un procédé utilisant un moulin colloïdal, un procédé utilisant un mélangeur statique, un procédé utilisant un micro mélangeur, un procédé utilisant une pale cadre, un procédé utilisant un extrudeur-malaxeur, un procédé utilisant une membrane poreuse, un procédé utilisant un contrôle du mûrissement, un procédé utilisant un gonflement d'un latex "templating agent", un procédé utilisant les instabilités de Rayleigh, et un procédé utilisant un fractionnement d'émulsion polydisperse.

**30.** Procédé selon la revendication 26 ou 27, **caractérisé en ce que** le processus conduisant à obtenir des particules est adapté à l'obtention de particules d'une taille moyenne allant d'environ 150 nm à environ 1 $\mu$m.

**31.** Procédé selon la revendication précédente, **caractérisé en ce que** ledit processus est choisi parmi un procédé développant un cisaillement et un procédé mettant en jeu une inversion de phase.

**32.** Procédé selon l'une quelconque des revendications 26 à 31, **caractérisé en ce que** la température de l'étape d'émulsification est supérieure à 40 °C.

**33.** Particules ou dispersion de particules selon l'une quelconque des revendications 1 à 24, susceptibles d'être obtenues selon le procédé tel que défini selon l'une quelconque des revendications 26 à 32.

**34.** Utilisation de particules selon l'une quelconque des revendications 1 à 19 et/ou d'au moins une dispersion selon l'une quelconque des revendications 20 à 24 dans une composition cosmétique destinée à la photoprotection.

**35.** Procédé de maquillage et/ou de soin non thérapeutique de la peau, comprenant au moins une étape d'application sur celle-ci d'au moins une composition comprenant des particules telles que définies selon l'une quelconque des revendications 1 à 19 et 33 et/ou d'au moins une dispersion telle que définie selon l'une quelconque des revendications 20 à 24.

**36.** Utilisation de particules selon l'une quelconque des revendications 1 à 19 et 33 et/ou d'au moins une dispersion selon l'une quelconque des revendications 20 à 24 pour la fabrication d'une composition destinée à la protection de la peau et/ou des cheveux contre les effets néfastes des rayonnements UV, en particulier le rayonnement solaire.

Office européen des brevets

# RAPPORT DE RECHERCHE EUROPEENNE

Numéro de la demande
EP 06 30 0476

## DOCUMENTS CONSIDERES COMME PERTINENTS

| Catégorie | Citation du document avec indication, en cas de besoin, des parties pertinentes | Revendication concernée | CLASSEMENT DE LA DEMANDE (IPC) |
|---|---|---|---|
| Y | EP 1 331 000 A (L'OREAL)<br>30 juillet 2003 (2003-07-30)<br>* le document en entier *<br>----- | 1-36 | INV.<br>A61K8/04<br>A61Q17/00<br>A61Q17/04<br>A61K8/891<br>A61K8/81<br>A61K8/88<br>A61K8/37<br>A61K8/40 |
| Y | WO 02/092043 A (UNILEVER PLC; UNILEVER NV; HINDUSTAN LEVER LIMITED)<br>21 novembre 2002 (2002-11-21)<br>* page 4, ligne 22 - page 5, ligne 29 *<br>* page 9, ligne 1 - page 14, ligne 10 *<br>* page 18, ligne 26 - ligne 29 *<br>----- | 1-36 | |
| Y | US 2003/049282 A1 (ARONSON MICHAEL PAUL ET AL) 13 mars 2003 (2003-03-13)<br>* alinéa [0033] - alinéa [0069] *<br>* alinéa [0079] *<br>----- | 1-36 | |
| Y | US 6 126 948 A (SIMONNET ET AL)<br>3 octobre 2000 (2000-10-03)<br>* colonne 3, ligne 8 - colonne 4, ligne 51; exemple 2 *<br>----- | 1-36 | |
| Y | US 5 939 054 A (MSIKA ET AL)<br>17 août 1999 (1999-08-17)<br>* colonne 1, ligne 32 - colonne 2, ligne 61 *<br>----- | 1-36 | **DOMAINES TECHNIQUES RECHERCHES (IPC)**<br>A61K |
| Y | US 5 733 531 A (MITCHNICK ET AL)<br>31 mars 1998 (1998-03-31)<br>* colonne 4, ligne 15 - colonne 6, ligne 16 *<br>* colonne 14, ligne 40 - colonne 15, ligne 17 *<br>* revendications *<br>----- | 1-36 | |
| A | WO 95/28912 A (SUNSMART, INC; SUBMICRO ENCAPSULATION TECHNOLOGIES, INC)<br>2 novembre 1995 (1995-11-02)<br>* page 8, ligne 21 - page 9, ligne 27 *<br>-----<br>-/-- | 1-36 | |

Le présent rapport a été établi pour toutes les revendications

7

| Lieu de la recherche | Date d'achèvement de la recherche | Examinateur |
|---|---|---|
| Munich | 8 août 2006 | Irwin, L |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons
& : membre de la même famille, document correspondant

EPO FORM 1503 03.82 (P04C02)

Office européen des brevets

# RAPPORT DE RECHERCHE EUROPEENNE

Numéro de la demande
EP 06 30 0476

## DOCUMENTS CONSIDERES COMME PERTINENTS

| Catégorie | Citation du document avec indication, en cas de besoin, des parties pertinentes | Revendication concernée | CLASSEMENT DE LA DEMANDE (IPC) |
|---|---|---|---|
| A | US 6 521 217 B1 (LUTHER HELMUT ET AL)<br>18 février 2003 (2003-02-18)<br>* revendications *<br>----- | 1-36 | |
| | | | DOMAINES TECHNIQUES RECHERCHES (IPC) |

Le présent rapport a été établi pour toutes les revendications

| Lieu de la recherche | Date d'achèvement de la recherche | Examinateur |
|---|---|---|
| Munich | 8 août 2006 | Irwin, L |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P04C02)

7

## ANNEXE AU RAPPORT DE RECHERCHE EUROPEENNE RELATIF A LA DEMANDE DE BREVET EUROPEEN NO.

EP 06 30 0476

La présente annexe indique les membres de la famille de brevets relatifs aux documents brevets cités dans le rapport de recherche européenne visé ci-dessus.
Lesdits members sont contenus au fichier informatique de l'Office européen des brevets à la date du
Les renseignements fournis sont donnés à titre indicatif et n'engagent pas la responsabilité de l'Office européen des brevets.

08-08-2006

| Document brevet cité au rapport de recherche | | Date de publication | Membre(s) de la famille de brevet(s) | | | Date de publication |
|---|---|---|---|---|---|---|
| EP 1331000 | A | 30-07-2003 | FR | 2834883 | A1 | 25-07-2003 |
| | | | JP | 2003212744 | A | 30-07-2003 |
| WO 02092043 | A | 21-11-2002 | BR | 0209897 | A | 08-06-2004 |
| | | | CN | 1543336 | A | 03-11-2004 |
| | | | CZ | 20033110 | A3 | 17-03-2004 |
| | | | EP | 1390006 | A2 | 25-02-2004 |
| | | | HU | 0400609 | A2 | 29-11-2004 |
| | | | JP | 2005508286 | T | 31-03-2005 |
| | | | MX | PA03010473 | A | 09-03-2004 |
| | | | PL | 367227 | A1 | 21-02-2005 |
| US 2003049282 | A1 | 13-03-2003 | US | 2005244356 | A1 | 03-11-2005 |
| | | | ZA | 200308135 | A | 20-10-2004 |
| US 6126948 | A | 03-10-2000 | CA | 2230097 | A1 | 13-09-1998 |
| | | | DE | 69800032 | D1 | 02-12-1999 |
| | | | DE | 69800032 | T2 | 20-07-2000 |
| | | | EP | 0864320 | A1 | 16-09-1998 |
| | | | ES | 2140988 | T3 | 01-03-2000 |
| | | | FR | 2760641 | A1 | 18-09-1998 |
| | | | JP | 3095726 | B2 | 10-10-2000 |
| | | | JP | 10298051 | A | 10-11-1998 |
| US 5939054 | A | 17-08-1999 | AT | 201583 | T | 15-06-2001 |
| | | | DE | 69613108 | D1 | 05-07-2001 |
| | | | DE | 69613108 | T2 | 31-01-2002 |
| | | | DK | 814759 | T3 | 17-09-2001 |
| | | | EP | 0814759 | A1 | 07-01-1998 |
| | | | ES | 2159356 | T3 | 01-10-2001 |
| | | | FR | 2731615 | A1 | 20-09-1996 |
| | | | WO | 9628136 | A1 | 19-09-1996 |
| | | | GR | 3036389 | T3 | 30-11-2001 |
| | | | JP | 11501651 | T | 09-02-1999 |
| | | | PT | 814759 | T | 30-11-2001 |
| US 5733531 | A | 31-03-1998 | AUCUN | | | |
| WO 9528912 | A | 02-11-1995 | AU | 2425195 | A | 16-11-1995 |
| | | | CA | 2188166 | A1 | 02-11-1995 |
| | | | EP | 0756478 | A1 | 05-02-1997 |
| | | | JP | 10504520 | T | 06-05-1998 |
| US 6521217 | B1 | 18-02-2003 | AU | 757342 | B2 | 20-02-2003 |
| | | | AU | 4510999 | A | 10-01-2000 |
| | | | BR | 9911414 | A | 20-03-2001 |

EPO FORM P0460

Pour tout renseignement concernant cette annexe : voir Journal Officiel de l'Office européen des brevets, No.12/82

**ANNEXE AU RAPPORT DE RECHERCHE EUROPEENNE RELATIF A LA DEMANDE DE BREVET EUROPEEN NO.** EP 06 30 0476

La présente annexe indique les membres de la famille de brevets relatifs aux documents brevets cités dans le rapport de recherche européenne visé ci-dessus.
Lesdits members sont contenus au fichier informatique de l'Office européen des brevets à la date du
Les renseignements fournis sont donnés à titre indicatif et n'engagent pas la responsabilité de l'Office européen des brevets.

08-08-2006

| Document brevet cité au rapport de recherche | Date de publication | Membre(s) de la famille de brevet(s) | Date de publication |
|---|---|---|---|
| US 6521217 B1 | | CN 1306417 A | 01-08-2001 |
| | | DE 69907454 D1 | 05-06-2003 |
| | | DE 69907454 T2 | 18-03-2004 |
| | | WO 9966896 A1 | 29-12-1999 |
| | | ES 2196816 T3 | 16-12-2003 |
| | | ID 26827 A | 15-02-2001 |
| | | JP 2002518428 T | 25-06-2002 |

EPO FORM P0460

Pour tout renseignement concernant cette annexe : voir Journal Officiel de l'Office européen des brevets, No.12/82

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description


- US 6737394 B
- WO 02092043 A
- EP 0375520 A
- EP 1331000 A
- US 20040042980 A
- US 20040247549 A
- FR 0302809
- EP 951897 A
- US 5156911 A
- WO 0119333 A
- US 5736125 A
- US 5519063 A
- EP 0550745 A
- US 5783657 A
- US 3645705 A
- US 3148125 A
- US 5500209 A
- US 5756082 A
- EP 0497144 A
- WO 9842298 A
- US 6225390 B
- US 6160054 A
- US 6174968 B
- US 5468477 A
- US 5725882 A
- US 4367390 A
- EP 863145 A
- EP 517104 A
- EP 570838 A
- EP 796851 A
- EP 775698 A
- EP 878469 A
- EP 933376 A
- EP 507691 A
- EP 507692 A
- EP 790243 A
- EP 944624 A
- EP 669323 A
- US 2463264 A
- US 5237071 A
- US 5166355 A
- GB 2303549 A
- DE 19726184
- EP 893119 A
- WO 9304665 A
- DE 19855649
- EP 0967200 A
- DE 19746654
- DE 19755649
- EP 1008586 A
- EP 1133980 A
- EP 133981 A
- GB 2206339 A
- US 6541018 B
- US 6335022 B
- US 6375960 B
- US 6689371 B
- US 6461625 B
- US 6413527 B
- US 6274150 B
- US 6464990 B
- US 6120778 A
- EP 1466588 A
- US 20030138465 A
- US 20040214913 A
- US 20030147832 A
- US 20020198328 A
- FR 2856923
- FR 2747321
- US 5558820 A
- US 5688842 A
- WO 0240574 A
- US 5326484 A
- US 6160061 A
- EP 719087 A


### Littérature non-brevet citée dans la description


- **S. NOJIMA.** Melting behavior of poly(Σ -caprolactone)-block-polybutadiène copolymers. *Macromolécules,* 1999, vol. 32, 3727-3734
- **B. BOUTEVIN et al.** Study of morphological and mechanical properties of PP/PBT. *Polymer Bulletin,* 1995, vol. 34, 117-123
- **P. RANGARAJAN et al.** Morphology of semi-crystalline block copolymers of ethylene- (ethylene-alt-propylene. *Macromolecules,* 1993, vol. 26, 4640-4645

- **P. RICHTER et al.** Polymer agregates with crystalline cores : the system poly(ethylene)- poly(ethylene-propylene. *Macromolécules,* 1997, vol. 30 (25), 1053-1068
- **I.W. HAMLEY.** Cristallization in block copolymers. *Advances in Polymer Science,* 1999, vol. 148, 113-137
- *Cosmetics & Toiletries,* Février 1990, vol. 105, 53-64
- **B.L. DIFFEY.** *J. Soc. Cosmet. Chem.,* 1989, vol. 40, 127-133
- **WEITZ.** *Langmuir,* 2000, vol. 16, 347-351
- **BIBETTE.** *J. Coll. Int. Sci.,* 1991, vol. 147 (2), 474-478